# EUROPEAN PATENT SPECIFICATION

(11) **EP 2 316 346 B1**
(45) Date of publication and mention of the grant of the patent: **27.12.2017**
(21) Application number: 09824817.2
(22) Date of filing: 05.11.2009
(51) Int. Cl.: A61B 17/04, A61B 1/00, A61B 1/012

(54) **SUTURE DEVICE AND SUTURE SYSTEM HAVING TWO ENDOSCOPES**
NAHTVORRICHTUNG UND NAHTSYSTEM MIT ZWEI ENDOSKOPEN
DISPOSITIF DE SUTURE ET SYSTÈME DE SUTURE POURVU DE DEUX ENDOSCOPES

(30) Priority: 06.11.2008 US 265790
(43) Date of publication of application: 04.05.2011
(73) Proprietor: Olympus Corporation, Tokyo 192-8507 (JP)
(72) Inventor: SUZUKI, Takayuki, Tokyo 151-0072 (JP); YAMAMOTO, Tetsuya, Tokyo 151-0072 (JP); TAKAHASHI, Shinji, Tokyo 151-0072 (JP); SUZUKI, Satoko, Tokyo 151-0072 (JP); KOGASAKA, Takahiro, Tokyo 151-0072 (JP)
(74) Representative: Gunzelmann, Rainer
(86) International application number: PCT/JP2009/068896
(87) International publication number: WO 2010/053118

(56) References cited:
- EP-A1- 1 938 760
- WO-A1-2006/098155
- WO-A1-2007/037326
- WO-A2-2006/005061
- JP-A- 8 033 635
- JP-A- 8 112 252
- JP-A- 2006 116 356
- JP-A- 2007 229 472
- JP-T- 2007 532 262
- JP-T- 2008 531 207
- US-A1- 2005 085 691
- US-A1- 2005 090 709
- US-A1- 2006 106 286
- US-A1- 2007 185 503
- US-A1- 2007 293 720

## Description

### TECHNICAL FIELD

The present invention relates to a suturing device inserted into a body cavity, and more particularly, to a suturing device used to suture a perforation formed in a hollow organ such as a stomach or an intestine by means of a suturing thread, of which an end portion is mounted with an anchor, and a suturing system including the suturing device.

### BACKGROUND ART

Conventionally, there is known a suturing device which uses a suturing thread, of which an end portion is mounted with an anchor, for the purpose of suturing a perforation or a laceration formed in a hollow organ such as a stomach or an intestine (for example, see Patent Document 1). In this suturing device, the suturing operation is carried out in such a manner that the anchor released from a distal end of a needle is locked on a tissue in the vicinity of a perforation inside or outside the tissue, and the suturing thread is pulled by means of a stopper or the like so as to pull the tissues locked by the anchors.
[Patent Document 1] PCT International Publication No. WO2007-37326 discloses a suturing system according to the preamble of appended claim 1.
[Patent Document 2] US Patent Application Publication No. US 2007/0185503 A1 Patent Document 2 discloses a suture instrument comprising a first and second endoscope. The first endoscope supports needle forceps and a fastening tool and comprises a first and a second channel. The second endoscope supports gripping forceps.
[Patent Document 3] US Patent Application Publication No. US 2007/0293720 A1 Patent Document 3 discloses an endoscope with an imaging catheter assembly. The endoscope comprises an insertion tube with an auxiliary imaging device connected to the endoscope via a link. The insertion tube can further be provided with a wire loop for excising a polyp.

### DISCLOSURE OF THE INVENTION

### PROBLEMS TO BE SOLVED BY THE INVENTION

However, in the known suturing device, since the needle tube is formed in a linear shape, it is difficult to visibly recognize the position of the distal end portion of the needle tube when it is inserted into the tissue by means of the endoscope device, and the distal end of the needle tube is not visibly recognized enough for a reliable releasing operation of the anchor.

The present invention was devised in view of the above circumstances, and has as an object the provision of a suturing device which can visibly recognize the position of the distal end portion of the needle tube and a suturing system including the suturing device.

### MEANS FOR SOLVING THE PROBLEMS

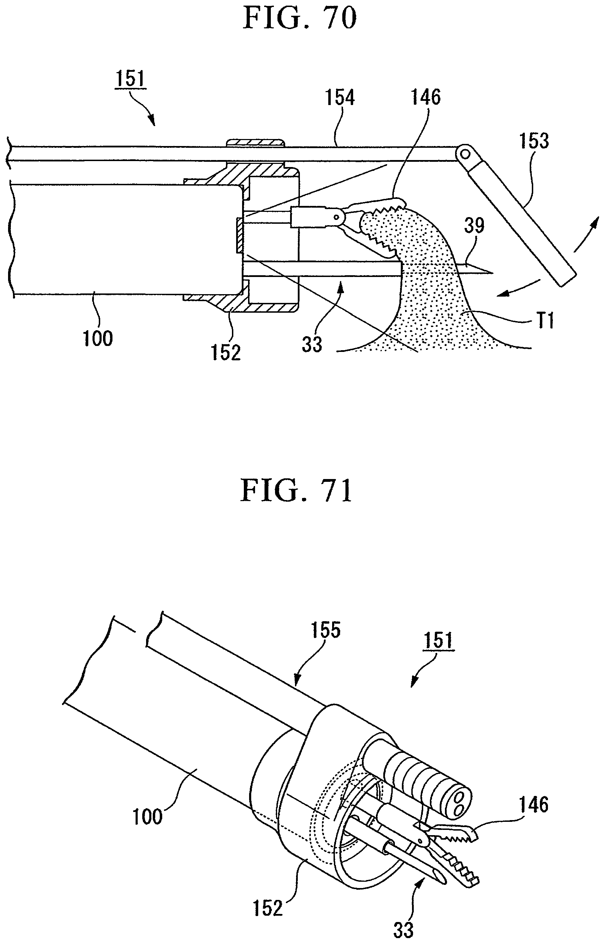

The invention is defined in appended claim 1. An embodiment of the invention is disclosed in Fig. 71-72 and the corresponding description.

### ADVANTAGEOUS EFFECTS OF THE INVENTION

According to the present invention, it is possible to observe the needle tube by means of the endoscope device when the needle tube is inserted into the target tissues or the like and after the needle tube has penetrated the tissue. It is possible to check that the distal end of the needle tube comes out from the target tissue or the like by means of the endoscope device. Also, it is possible to release the anchors while observing them by means of the endoscope device. Accordingly, it is possible to accurately carry out the suturing operation by reliably locking the suturing unit on the treatment target tissues.

### BRIEF DESCRIPTION OF THE DRAWINGS

FIG. 1 is a view showing a suturing device according to a first embodiment.
FIG 2 is a view showing a suturing unit used in the suturing device.
FIG 3 is a partially enlarged sectional view showing a distal end part of the suturing device.
FIG 4 is a view showing an operation upon using the suturing device.
FIG 5 is a view showing an operation upon using the suturing device.
FIG 6 is a view showing an operation upon using the suturing device.
FIG 7 is an overall view showing a configuration of the suturing device according to a second embodiment.
FIG 8A is a partially enlarged sectional view showing a distal end part of a suturing mechanism of the suturing device.
FIG 8B is a partially enlarged sectional view showing an operation part of the suturing mechanism.
FIG 9 is a view showing operations upon using the suturing device.
FIG 10 is a view showing operations upon using the suturing device.
FIG 11 is a view showing operations upon using the suturing device.
FIG 12 is a view showing operations upon using the suturing device.
FIG 13 is a view showing operations upon using the suturing device.
FIG 14 is a view showing operations upon using the suturing device.
FIG 15 is a view showing operations upon using the suturing device.
FIG 16 is a view showing a distal end part of the suturing device according to a third embodiment.
FIG 17 is an enlarged sectional view showing the distal end part.
FIG 18 is a view showing the operation part of the suturing device.
FIG 19 is a sectional view showing the operation part.
FIG 20 is a view showing operations upon using the suturing device.
FIG 21 is a view showing operations upon using the suturing device.
FIG 22 is a view showing operations upon using the suturing device.
FIG 23 is a view showing operations upon using the suturing device.
FIG 24 is a view showing operations upon using the suturing device.
FIG 25 is a view showing operations upon using the suturing device.
FIG 26 is a view showing operations upon using the suturing device.
FIG 27 is a view showing operations upon using the suturing device.
FIG 28 is a view showing operations upon using the suturing device.
FIG 29 is a view showing operations upon using the suturing device.
FIG 30 is a view showing operations upon using the suturing device.
FIG 31 is an enlarged sectional view showing a distal end part of the suturing device according to a modified example of the embodiment.
FIG 32 is a view showing operations upon using the suturing device.
FIG 33 is a view showing operations upon using the suturing device.
FIG 34 is a view showing operations upon using the suturing device.
FIG 35 is a view showing operations upon using the suturing device.
FIG 36 is a view showing a needle tube of the suturing device according to another modified example of the embodiment.
FIG. 37 is an enlarged sectional view showing the needle tube.
FIG 38 is a view showing operations upon using the suturing device.
FIG 39 is a view showing operations upon using the suturing device.
FIG 40 is a view showing the needle tube of the suturing device according to still another embodiment.
FIG 41A is a sectional view taken along the line A-A of FIG 40.
FIG 41B is a sectional view taken along the line B-B of FIG 40.
FIG 41C is a sectional view taken along the line C-C of FIG 40.
FIG 42 is a view showing an example of an endoscope forceps of a suturing system according to a fourth embodiment.
FIG 43 is an enlarged view showing a distal end part of the endoscope forceps.
FIG 44 is an enlarged view showing a distal end part of the suturing system.
FIG 45 is a view showing operations upon using the suturing system.
FIG 46 is a view showing operations upon using the suturing system.
FIG 47 is a view showing operations upon using the suturing system.
FIG 48 is a view showing operations upon using the suturing system.
FIG 49 is a view showing a distal end part of the suturing system according to a fifth embodiment.
FIG 50 is a front view showing the distal end part of the suturing system.
FIG 51 is a view showing operations upon using the suturing system.
FIG 52 is a view showing operations upon using the suturing system.
FIG 53 is a view showing operations upon using the suturing system.
FIG 54 is a view showing operations upon using the suturing system.
FIG 55 is an overall view showing the suturing system according to a sixth embodiment.
FIG 56 is a view showing operations upon using the suturing system.
FIG 57 is a view showing operations upon using the suturing system.
FIG 58 is a view showing operations upon using the suturing system.
FIG 59 is a view showing a distal end part of the suturing system according to a seventh embodiment.
FIG 60 is a sectional view showing a puncture cap of the suturing system in an axial direction.
FIG 61 is a view showing operations upon using the suturing system.
FIG 62 is a view showing operations upon using the suturing system.
FIG 63 is a view showing operations upon using the suturing system.
FIG 64 is a view showing operations upon using the suturing system.
FIG 65 is an overall view showing the suturing system according to an eighth embodiment.
FIG 66 is a view showing operations upon using the suturing system.
FIG 67 is a view showing operations upon using the suturing system.
FIG 68 is a view showing operations upon using the suturing system.
FIG 69 is a view showing a distal end part of the suturing system according to a ninth embodiment.
FIG 70 is a view showing an operation upon using the suturing system.
FIG 71 is a view showing a distal end part of the suturing system according to the invention.
FIG 72 is a view showing an operation upon using the suturing system.
FIG 73 is a view showing the needle tube of the suturing device according to a tenth embodiment.
FIG 74 is a view showing a state where the needle tube is received in a sheath.
FIG 75 is a view showing an operation upon using the suturing device.
FIG 76 is a view showing an operation upon using the suturing device.
FIG 77 is a view showing the needle tube of the suturing device according to a modified example of the embodiment.
FIG 78A is a view showing the needle tube of the suturing device according to the modified example of the embodiment.
FIG 78B is a view showing a state where an anchor passes through the needle tube.

### EMBODIMENTS FOR CARRYING OUT THE INVENTION

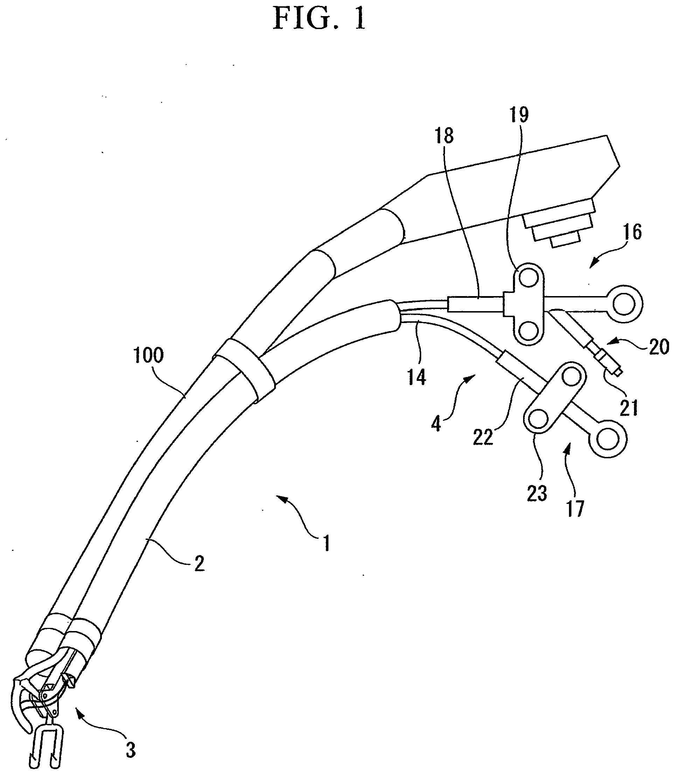

Hereinafter, a suturing device according to a first embodiment will be described with reference to FIGS. 1 to 5.

FIG 1 is a view showing a suturing device 1 according to this embodiment. The suturing device 1 is tied with an endoscope device 100 by means of a tape, or the like, so as to be inserted into a patient's body cavity.

The suturing device 1 includes an insertion part 2 which is inserted into a body, a distal end part 3 which is formed at the distal end portion of the insertion part 2 so as to carry out a suturing operation, and an operation part 4 which is provided on the side of the proximal end of the insertion part 2 so as to operate the distal end part 3.

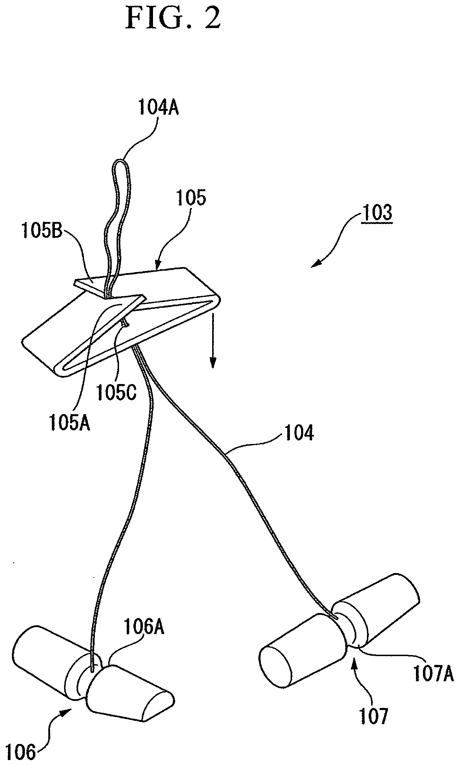

FIG. 2 is a view showing a suturing unit 103 mounted to the suturing device 1 so as to be received in the suturing device 1. The suturing unit 103 includes a suturing thread 104, a stopper 105 through which the suturing thread 104 is inserted, and first and second bar-shaped anchors 106 and 107 which are mounted to both ends of the suturing thread 104. The anchors 106 and 107 are respectively provided with engagement grooves 106A and 107A which are formed in the whole circumference of a part of outer surfaces thereof.

The stopper 105 is formed in such a manner that a plate-shaped member made of metal or a resin such as a biodegradable resin is bent so as to allow left and right end portions 105A and 105B to face each other and the end portions 105A and 105B engage with each other.

A hole 105C is formed in the vicinity of a center portion of the stopper 105 in a horizontal direction. The suturing thread 104 bent at a center point 104A is inserted through the hole 105C from a surface opposite to the end portions 105A and 105B and is disposed so as to pass through a gap between the end portions 105A and 105B engaging with each other. An operation upon using the stopper 105 will be described below.

The insertion part 2 is a tubular member having flexibility. A wire used to operate the distal end part 3 or a pusher used to release the anchors 106 and 107 of the suturing unit 103 is inserted therethrough.

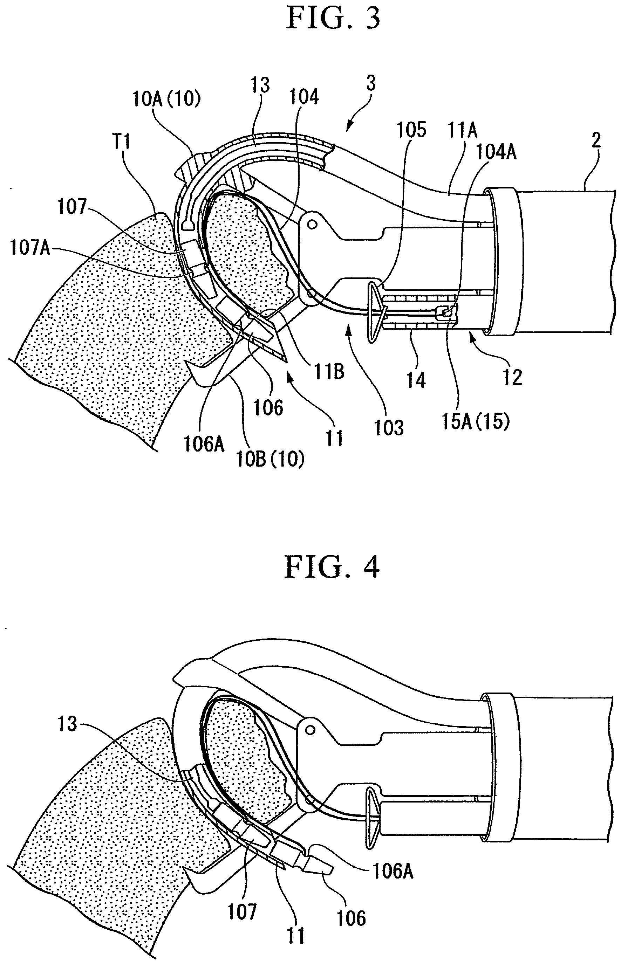

FIG 3 is a partially enlarged sectional view showing the distal end part 3. The distal end part 3 includes a pair of jaws 10 which grip a tissue of a treatment target portion (hereinafter, referred to as "target tissue"), a needle tube 11 which punctures the target tissue so as to release the anchors 106 and 107 therefrom, and a stopper support portion 12 by which the stopper 105 of the suturing unit 103 is supported.

The jaws 10 include a first upper jaw 10A and a second lower jaw 10B shown in FIG 3, and are capable of gripping the target tissue by closing the upper and lower jaws so as to allow mutually facing surfaces (gripping surfaces) to be close to each other. A wire (not shown) used to open or close the jaw 10 is connected to the operation part 4 via the insertion part 2.

The needle tube 11 is configured as a hollow member having a sharp distal end. As shown in FIG 3, the distal end portion of the needle tube 11 is formed in a hook shape so as to be curvedly folded back toward the distal end of the insertion part 2, and is integrally fixed to the first jaw 10A so as to pass through the gripping surface of the first jaw 10A. Accordingly, the needle tube 11 is interlocked with the open/close operation of the jaw 10 and moves so as to be rotated about the proximal end of the first jaw 10A.

In order to carry out the above-described movement, it is desirable that a base portion 11A of the needle tube 11 protruding from the insertion part 2 have constant flexibility. Such flexibility can be ensured in such a manner that the whole part of the needle tube 11 is made of super-elastic metal such as nickel titanium or a region of the proximal end portion rather than the base portion 11A is made of a resin or the like.

The anchors 106 and 107 of the suturing unit are received in an inner cavity of the needle tube 11. The engagement grooves 106A and 107A of the anchors respectively engage with engagement protrusions (not shown) protruding to the inside of the needle tube 11, thereby preventing the anchors 106 and 107 from being erroneously released or naturally coming off.

The suturing thread 104 of the suturing unit 103 is drawn to the outside of the needle tube 11 from a groove 11B formed in the inner peripheral side of the hook shape of the needle tube 11. A pusher 13 used to release the anchors 106 and 107 is inserted through the proximal end side rather than the anchors 106 and 107. The pusher 13 extends up to the operation part 4 via the insertion part 2.

Since the anchors 106 and 107 are received in the curved inner cavity of the needle tube 11 as shown in FIG 3, it is desirable to form the anchors in a tapered shape in which the diameter gradually decreases toward both end portions in a longitudinal direction, where the both end portions are hardly caught by the inner wall of the needle tube 11. Additionally, when the anchors are made of the above-described super-elastic metal or biodegradable plastic so as to have flexibility, it is desirable in that the movement and the releasing in the needle tube 11 are smooth. In the same manner, the pusher 13 is also formed to have flexibility since it advances and retracts in the curved inner cavity.

The stopper support portion 12 includes a sheath 14 which is inserted through the insertion part and a lock member 15 which is inserted through the sheath 14. The sheath 14 is configured as a tubular member having flexibility so as to freely advance and retract in an axial direction of the insertion part 2. The lock member 15 includes a hook 15A formed at the distal end portion thereof and extends up to the operation part 4.

As shown in FIG 3, the suturing thread 104 drawn out from the groove 11B of the needle tube 11 is drawn into the inside of the sheath 14. Then, the center point 104A where the suturing thread 104 is bent is locked in the hook 15A of the lock member 15, and the stopper 105 comes into contact with the distal end portion of the sheath 14 and is supported thereby.

Returning to FIG 1, the operation part 4 includes a first operation part 16 which is used to operate the jaws 10 and the pusher 13 and a second operation part 17 which is used to operate the stopper support portion 12.

The first operation part 16 includes a body 18, a slider 19 which is mounted to the body 18 in a freely sliding manner in an axial direction, and a pusher operation portion 20 of which one end portion is mounted to the body 18. The slider 19 is connected to the end portion of the wire connected to the jaws 10. When the slider 19 is slid on the body 18, the jaws 10 are opened or closed.

The pusher operation portion 20 is configured as a cylindrical member and extends toward the proximal end of the body 18 while forming a predetermined angle with respect to the body 18. The proximal end portion of the pusher 13 which has passed through the inside of the insertion part 2 and the body 18 is inserted through the pusher operation portion 20 in a freely advancing and retracting manner, and a handle 21 is mounted to the end portion of the protruding pusher 13. When a user advances the pusher 13 toward the pusher operation portion 20 by means of the handle 21, it is possible to release the anchors 106 and 107 from the needle tube 11.

The second operation part 17 includes a body 22 and a slider 23 which is mounted to the body 22 in a freely sliding manner in an axial direction. The body 22 is connected to the proximal end of the sheath 14 inserted through the insertion part 2. When the body 22 advances or retracts with respect to the insertion part 2, it is possible to change a protrusion length of the sheath 14 from the distal end of the insertion part 2. The slider 23 is connected to the proximal end of the lock member 15 inserted through the sheath 14. When the slider 23 is slid on the body 22, it is possible to change a positional relationship between the sheath 14 and the lock member 15 in a longitudinal direction.

An operation upon using the suturing device 1 with the above-described configuration will be described.

First, a user inserts the suturing device 1 into a body cavity of a patient or the like so that the distal end part 3 moves up to a position in the vicinity of a stomach wall or the like having a treatment target portion such as a perforation or a laceration. At this time, first, an endoscope device 100 is inserted into the body cavity, and the proximal end portion of the endoscope device 100 is inserted through a known overtube (not shown). Subsequently, the overtube is inserted into the body cavity by using the endoscope device 100 as a guide, and the endoscope device 100 is once taken out. Subsequently, as shown in FIG 1, the endoscope device 100 and the suturing device 1 are tied together by means of a tape, a band or the like, and are inserted into the overtube so as to be inserted into the body cavity, thereby smoothly introducing the distal end part 3 having a complex shape up to a position in the vicinity of the target tissue.

The user opens the jaws 10 by operating the slider 19 of the first operation part 16, moves the jaws to be close to one target tissue T1 of the treatment target portion, and then closes the jaws 10. Then, as shown in FIG 3, the target tissue T1 is gripped by the jaws 10 and the sharp distal end portion of the needle tube 11 passing through the gripping surface of the first jaw 10A is inserted into the target tissue T1 at the same time. When the user further closes the jaws 10, the distal end of the needle tube 11 penetrates the target tissue T1 while passing through a gap between both bridges of the second jaw 10B.

Subsequently, when the user advances the pusher 13 by operating the pusher operation portion 20, as shown in FIG 4, the pusher pushes the anchors 106 and 107 toward the distal end of the needle tube, and the first anchor 106 disposed on the distal end side is first released from the needle tube 11. The user can recognizes a fact that the first anchor 106 is released by means of the sense of touch (click-feeling) occurring when the engagement protrusion formed inside the needle tube 11 passes over the engagement groove 106A of the first anchor 106.

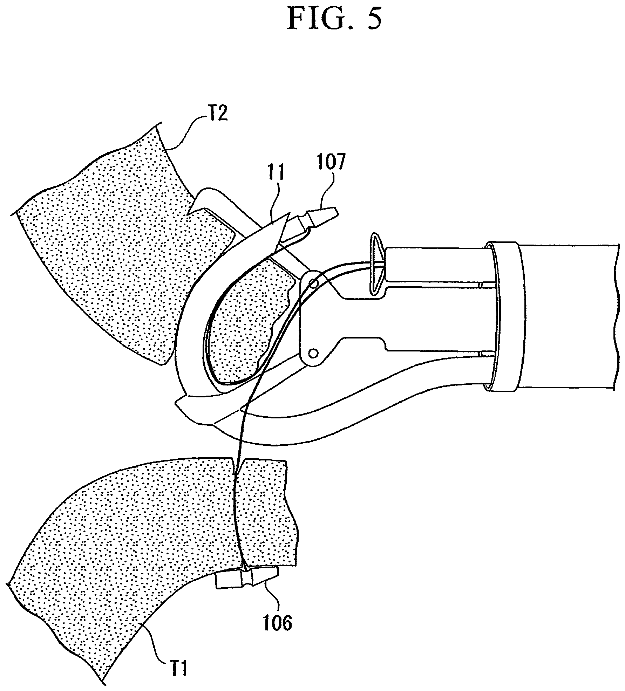

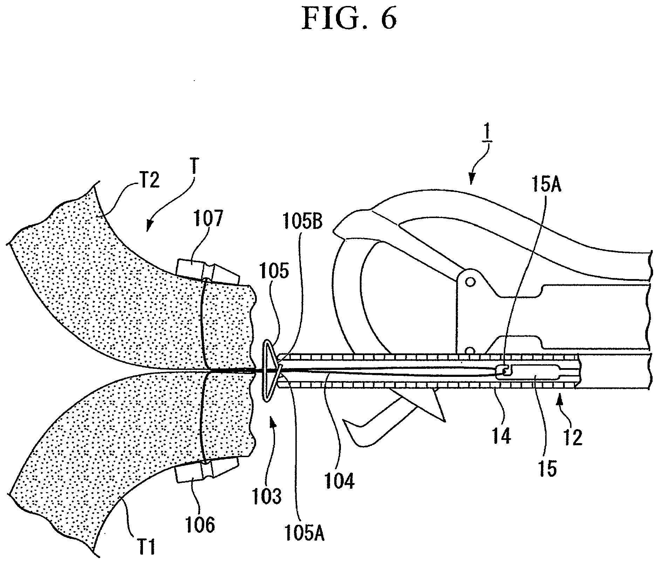

In the same manner, as shown in FIG 5, the user releases the second anchor 107 to lock it on the other target tissue T2 of the treatment target portion. After the needle tube 11 is taken out from the target tissue T2, the user pushes the body 22 of the second operation part 17 into the insertion part 2 while maintaining a positional relationship between the body 22 and the slider 23. Then, as shown in FIG 6, the sheath 14 of the stopper support portion 12 protrudes from the insertion part 2.

When the sheath 14 protrudes from the insertion part 2, the suturing thread 104 of the suturing unit 103 locked in the hook 15A of the lock member 15 is received in the sheath 14, and only the suturing thread 104 is received in the sheath 14 in a state where the stopper 105 still comes into contact with the distal end surface of the sheath 14, thereby shortening the distance between the stopper 105 and the anchors 106 and 107.

Since the anchors 106 and 107 are locked on the tissues T1 and T2, respectively, as shown in FIG 6, as the stopper 105 moves closer to the anchors 106 and 107, the target tissues T1 and T2 are drawn toward the suturing device 1 together with the anchors 106 and 107 so as to come into close contact with each other. In this manner, the suturing operation of the treatment target portion T is carried out.

At this time, although the engagement between the end portions 105A and 105B of the stopper 105 is loosened when the suturing thread 104 moves toward the center point 104A so as to be received in the sheath 14, the end portions 105A and 105B more forcedly engage with each other by means of a force acting on the suturing thread 104 even if the suturing thread 104 tries to move toward the anchors 106 and 107, thereby prohibiting the movement of the suturing thread in the corresponding direction. That is, since the stopper 105 moves only in a direction toward the anchors 106 and 107 and is not moved in an opposite direction, the suturing degree of the treatment target portion T is not loosened or released.

After the suturing operation ends, the user retracts the sheath 14. Subsequently, the user advances the lock member 15 with respect to the sheath 14 by operating the slider 23 so as to protrude from the sheath 14. Subsequently, the user releases the engagement between the hook 15A and the suturing thread 104 while observing by means of the endoscope device 100 so as to separate the suturing unit 104 from the suturing device 1. In this manner, a series of treatments ends.

In the suturing device 1 according to this embodiment, the needle tube 11, from which the anchors 106 and 107 are released, is curved so as to pass through the gripping surface of the first jaw 10A of the pair of jaws 10 which are capable of opening and closing. Accordingly, when the needle tube 11 is inserted into the target tissues T1 and T2, it is possible to observe the needle tube by means of the endoscope device 100. Also, since the distal end of the needle tube 11 faces the insertion part 2 even after the needle tube 11 penetrates the tissue, it is possible to observe the needle tube by means of the endoscope device 100.

In the known suturing device, since the needle tube is formed in a linear shape, it is difficult to visibly recognize the position of the distal end portion of the needle tube when it is inserted into the tissue by means of the endoscope device, and the distal end of the needle tube is not visibly recognized enough for a reliable releasing operation of the anchor. In the suturing device 1 according to this embodiment, it is possible to check that the distal end of the needle tube 11 comes out from the target tissue T1 or the like by means of the endoscope device 100. Also, it is possible to release the anchors 106 and 107 while observing them by means of the endoscope device 100. Accordingly, it is possible to accurately carry out the suturing operation by reliably locking the suturing unit 103 on the treatment target tissues.

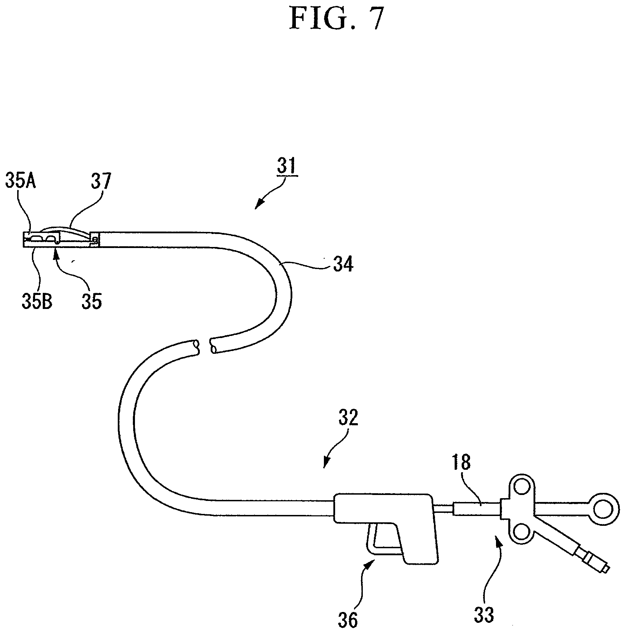

Next, a second embodiment will be described with reference to FIGS. 7 to 15. A suturing device 31 according to this embodiment is different from the above-described suturing device 1 in that the needle tube is introduced via a channel extending up to the jaws.

Note that, in the following description, the same reference numerals are given to the same components as those of the above-described embodiment, and the descriptions thereof will be omitted.

FIG 7 is an overall view showing a configuration of the suturing device 31. The suturing device 31 includes a gripping mechanism 32 which is provided with a pair of jaws and a suturing mechanism 33 which is inserted through the gripping mechanism 32.

A basic configuration of the gripping mechanism 32 is described in United States Patent Application, Publication No. 2006-0271073, and is briefly described as below.

The gripping mechanism 32 includes an insertion part 34 which is substantially equal to the insertion part 2, a pair of jaws 35 which is mounted to the distal end of the insertion part 34, and a handle 36 which is used to open or close the jaw 35.

A first upper jaw 35A is shorter than a second lower jaw 35B, and the proximal end of the first jaw 35A is rotatably supported to a position in the vicinity of a center portion of the second jaw 35B in a longitudinal direction. The proximal end of the second jaw 35B is rotatably supported to a position in the vicinity of the distal end of the insertion part 34.

The first jaw 35A is connected to a jaw open/close sheath 37 having flexibility. The jaw open/close sheath 37 in inserted through a channel provided in the insertion part 34 in a freely advancing and retracting manner, and has the proximal end connected to the handle 36. Accordingly, when a user advances or retracts the jaw open/close sheath 37 with respect to the insertion part 34 by operating the handle 36, the first jaw 35A is rotated, thereby opening or closing the jaws 35.

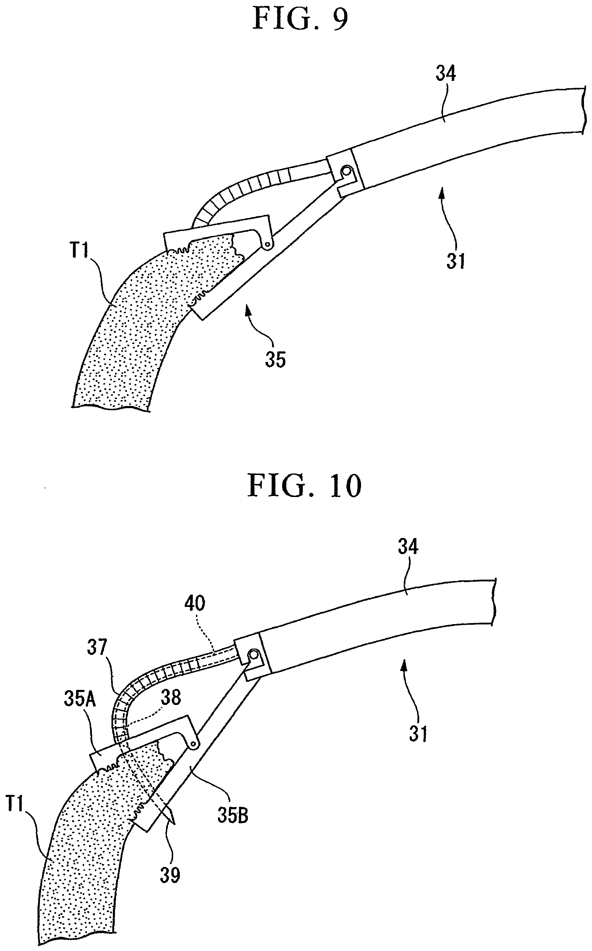

Since a through-hole 38 (see FIG 10) is formed through a gripping surface of the first jaw 35A facing the second jaw 35B, and the distal end of the jaw open/close sheath 37 is inserted through the through-hole 38 to be fixed thereto, the inner cavity of the jaw open/close sheath 37 extends up to the gripping surface of the first jaw 35A. The second jaw 35B is provided with a through-hole or a slit at a position corresponding to the through-hole 38, and the needle tube of the suturing mechanism 33 can pass through the gripping surface of the second jaw 35B during an operation described below.

Since the configuration of the suturing mechanism 33 is substantially equal to that of the suturing device 1 according to the first embodiment, the different points will be mainly described.

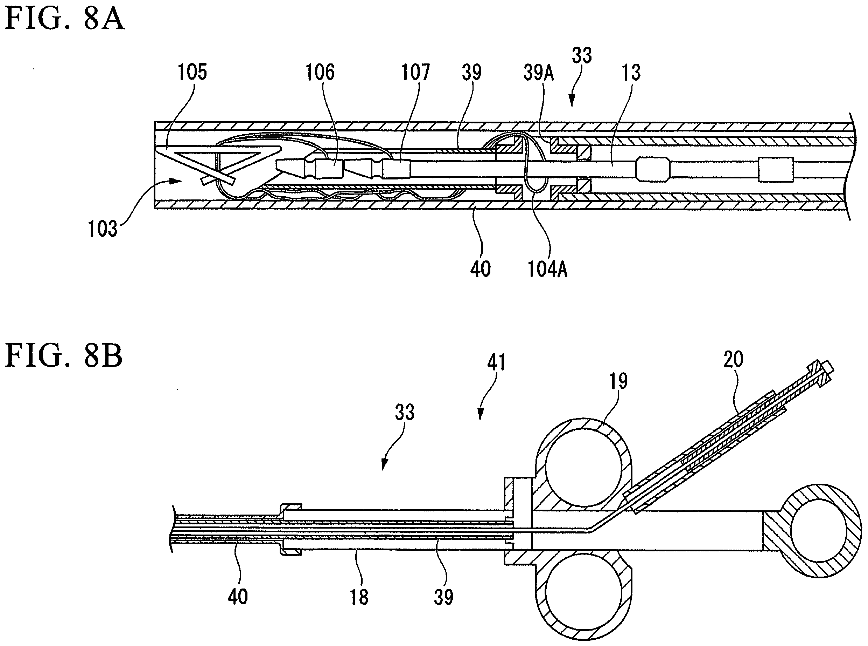

FIGS. 8A and 8B are partially enlarged sectional views respectively showing the distal end portion of the suturing mechanism 33 and an operation part 41 of the suturing mechanism 33. As shown in FIG. 8A, a needle tube 39 is formed in a linear shape. The needle tube 39 is inserted through a sheath 40 in a freely advancing and retracting manner, and the stopper 105 of the suturing unit 103 is also received in the inner cavity of the sheath 40. The center point 104A of the suturing thread 104 is inserted from a through-hole 39A formed in the needle tube 39 into the inner cavity of the needle tube 39 so as to be caught and rotated by the pusher 13.

As shown in FIG 8B, the proximal end of the sheath 40 is connected to the body 18 of the operation part 41, and the proximal end of the needle tube 39 is connected to the slider 19. The pusher operation portion 20 is mounted to the slider 19 and is slid on the body 18 together with the slider 19.

With the above-described configuration, when a user advances or retracts the slider 19 with respect to the body 18, it is possible to relatively move the needle tube 39 with respect to the sheath 40 and to protrude or depress the distal end of the needle tube 39 from the sheath 40. At this time, since the pusher operation portion 20 is slid together with the slider 19, a positional relationship between the needle tube 39 and the pusher 13 is not changed.

As shown in FIG 7, the suturing mechanism 33 is inserted from an insertion hole (not shown) provided in the handle 36 of the gripping mechanism 32 into the jaw open/close sheath 37 in a freely advancing and retracting manner. Accordingly, when the user pushes the body 18 toward the handle 36, it is possible to protrude the sheath 40 from the gripping surface of the first jaw 35A.

An operation upon using the suturing device 31 with the above-described configuration will be described.

First, the user ties the suturing device 31 and the endoscope device 100 together and introduces the jaws 35 up to a position in the vicinity of the treatment target portion. The timing when the suturing device 31 into the channel is inserted may be before or after the endoscope device 100 is inserted into the inner cavity of the body.

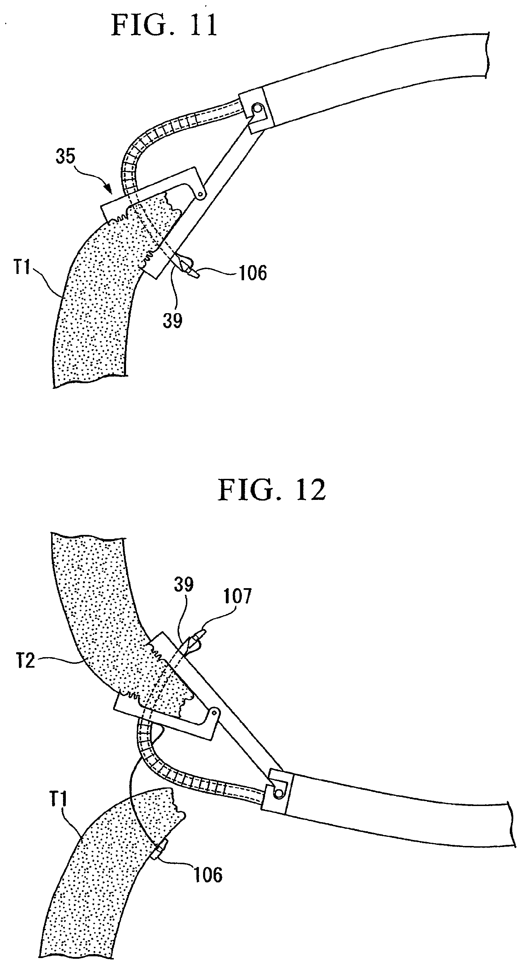

Subsequently, as shown in FIG 9, the user operates the handle 36 so that one target tissue T1 is gripped while being interposed between the jaws 35. Subsequently, the body 18 of the suturing mechanism 33 is advanced with respect to the insertion part 34, and the distal end of the sheath 40 moves to a position in the vicinity of the first jaw 35A. At the same time, the slide 19 is advanced with respect to the body 18 so as to penetrate the target tissue T1 by means of the needle tube 39 as shown in FIG 10, and the pusher 13 is operated so as to release the first anchor 106 from the needle tube 39 as shown in FIG 11, thereby locking the anchor on the target tissue T1.

At this time, since the distal end of the needle tube 39 faces the distal end portion of the endoscope device 100, the user is capable of easily observing a position of the distal end of the needle tube 39 or a state where the anchor is released by means of the endoscope device 100. After the first anchor 106 is released, the user retracts the needle tube 39 and releases the gripping state of the target tissue T1.

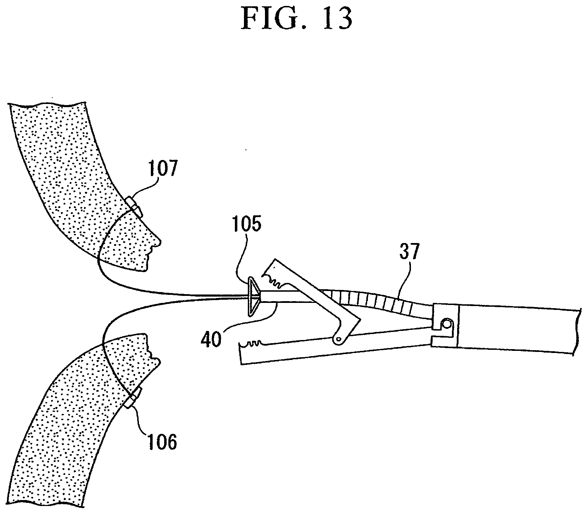

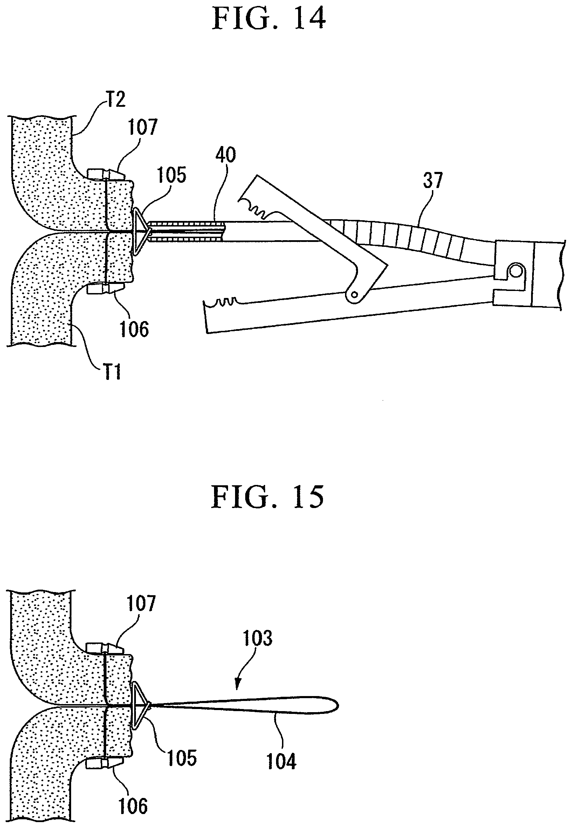

As shown in FIG 12, the user locks the second anchor 107 on the target tissue T2 in the same sequence. Then, after the stopper 105 is drawn to the outside of the sheath 40 as shown in FIG 13, the body 18 is advanced with respect to the jaw open/close sheath 37, and the stopper 105 is pushed by the sheath 40 as shown in FIG 14 so as to be moved toward the anchors 106 to 107. In this manner, the treatment target portion is tightly sutured.

When the user sufficiently retracts the pusher 13 with respect to the needle tube 39 by operating the pusher operation portion 20, the engagement between the suturing thread 104 and the pusher 13 is released, and the suturing unit 103 is separated from the suturing device 31 as shown in FIG 15.

In the case where the suturing operation is carried out in combination of the gripping mechanism 32 and the known suturing mechanism using a suturing unit in which an anchor is mounted to one end of a suturing thread and a stopper is not provided, it is necessary to insert the needle tube into the target tissues T1 and T2 while gripping both the target tissues T1 and T2 by the gripping mechanism 32 simultaneously. Since the two tissues are not easily gripped at the same time, the surgical technique is not easy.

Since the suturing unit 103 including the stopper 105 and two anchors 106 and 107 is used, it is possible to carry out the suturing operation of the treatment target tissues while reliably observing the distal end of the needle tube 39 in such a manner that the target tissues T1 and T2 are just separately gripped to lock the anchors thereon. Accordingly, it is possible to further easily and reliably carry out the suturing surgical technique.

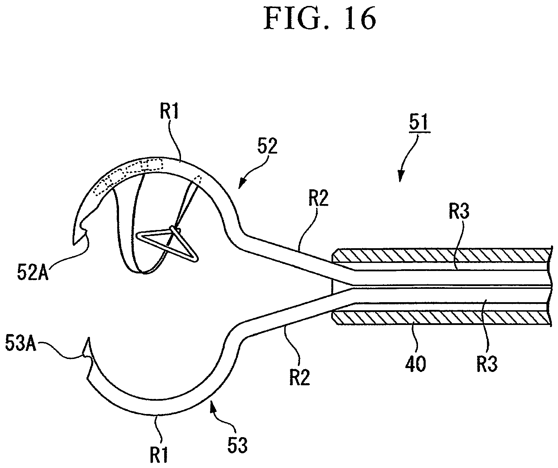

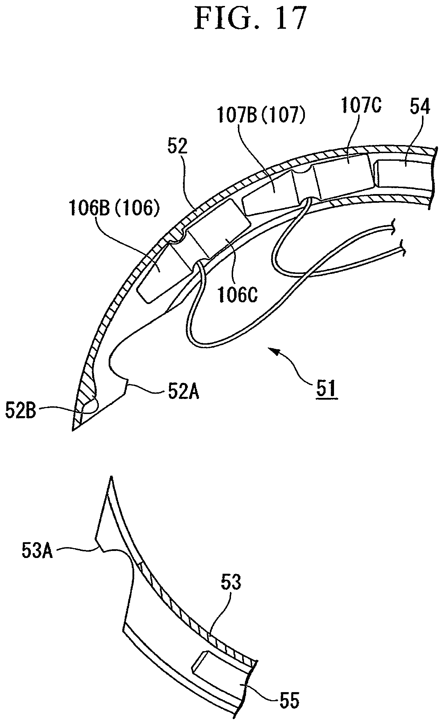

Next, a third embodiment will be described with reference to FIGS. 16 to 41C. A suturing device 51 according to this embodiment is different from the suturing devices according to the above-described embodiments in that two needle tubes are provided.

FIG 16 is a view showing the distal end portion of the suturing device 51. Two needle tubes, that is, a first needle tube 52 and a second needle tube 53 are inserted through the sheath 40. Each of the needle tubes 52 and 53 includes a first region R1 which is curved in a substantially circular-arc shape and is located on the distal end side, a second region R2 which is connected to the proximal end of the first region R1 and of which a portion closer to the distal end extends in a direction moving away from the axis of the sheath 40, and a third region R3 which is connected to the proximal end of the second region R2 and is connected to an operation part described below.

Although each of the needle tubes 52 and 53 is made of super-elastic metal as a whole, the first region R1 inserted into the tissue is made of rigid super-elastic metal having elasticity smaller than that of the second region R2, and the second region R2 is made of super-elastic metal having elasticity larger than that of the first region R1. Then, each of the needle tubes 52 and 53 is formed by means of welding or the like so as to integrally form the first region R1 and the second region R2 made of super-elastic metals having different elasticity. The third region R3 is made of the same super-elastic metal as that of the second region R2, and is formed by bending a boundary portion between the regions R2 and R3. Alternatively, the third region R3 may be made of flexible super-elastic metal having larger elasticity or a flexible resin, and may be bonded to the second region R2. In the case of the resin, for example, polyetheretherketone (PEEK) can be appropriately used.

As shown in FIG 16, the first regions R1 of the needle tubes 52 and 53 are convexly formed to the outside in a radial direction of the sheath 40 so as to face each other. The distal ends of the needle tubes 52 and 53 are respectively provided with engagement protrusions 52A and 53A which are capable of engaging with each other in a freely detaching manner. When both the engagement protrusions engage with each other, it is possible to form the first regions of the needle tubes 52 and 53 in a substantially annular shape. Additionally, a releasing protrusion 52B protrudes from the inner peripheral surface in the vicinity of the engagement protrusion 52A so as to release the engagement between the engagement protrusions 52A and 53A. An angle of an inclined surface on the side of the proximal end portion of the releasing protrusion 52B is set to be not more than taper angles on the side of the distal end portions 106B and 107B of the anchors 106 and 107. The taper angles on the side of the proximal end portions 106C and 107C of the anchors 106 and 107 are smaller than those on the side of the distal end portions 106B and 107B, and the diameter reduction on the side of the proximal end portions is smaller than that on the side of the distal end portions.

First and second pusher 54 and 55, which are equal to the pusher 13, are respectively inserted through the needle tubes 52 and 53 so as to extend up to an operation part 56.

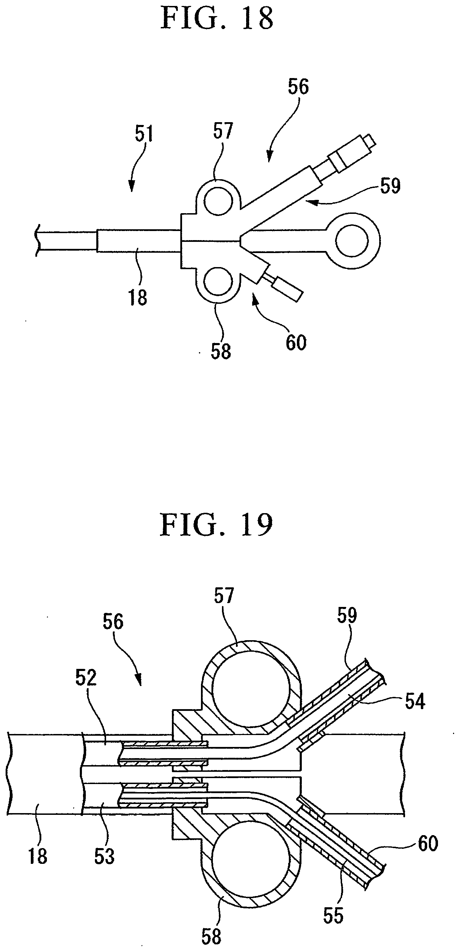

FIG 18 is a view showing the operation part 56 of the suturing device 51, and FIG 19 is a sectional view showing the operation part 56. The body 18 includes two sliders, that is, a first slider 57 which is connected to the proximal end of the first needle tube 52 and a second slider 58 which is connected to the proximal end of the second needle tube 53, where the two sliders are independently mounted to the body 18 in a freely sliding manner. The sliders 57 and 58 are respectively mounted with a first pusher operation portion 59 and a second pusher operation portion 60, and the proximal end portions of the first pusher 54 and the second pusher 55 are respectively inserted through the first pusher operation portion 59 and the second pusher operation portion 60.

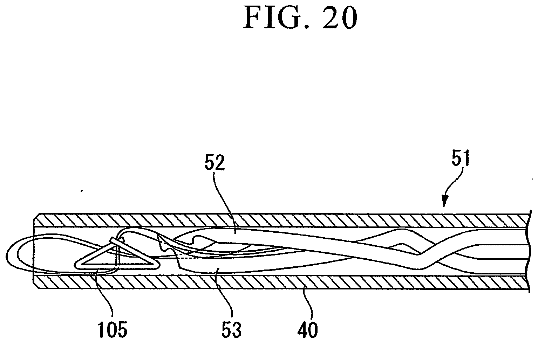

An operation upon using the suturing device 51 with the above-described configuration will be described with reference to FIGS. 20 to 30.

As shown in FIG 20, a user first inserts the sheath 40 into the channel of the endoscope device 100 in a state where the sliders 57 and 58 are retracted such that the stopper 105 and the needle tubes 52 and 53 are received in the sheath 40. Then, the endoscope device 100 is inserted into a patient's body so that the distal end of the endoscope device 100 moves up to a position in the vicinity of the treatment target portion.

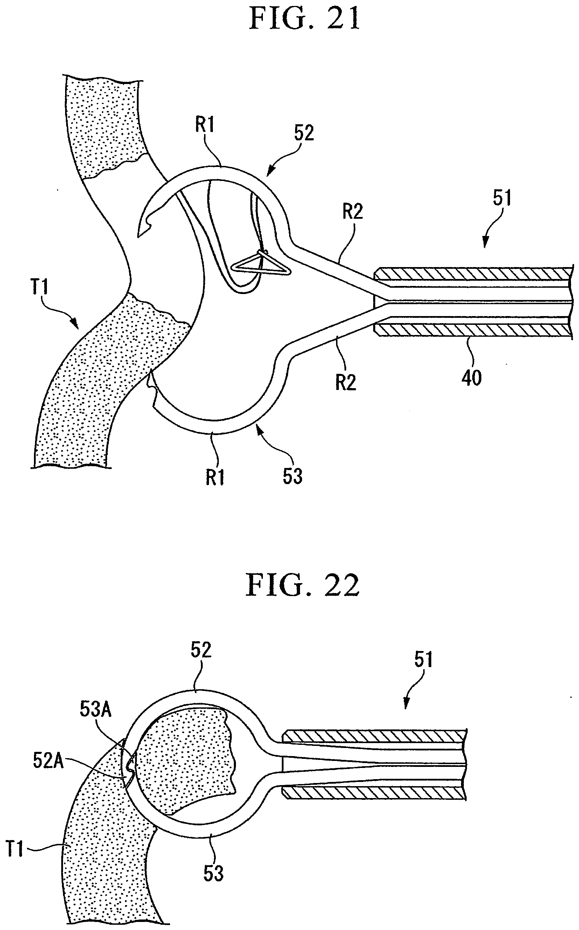

Subsequently, the user advances the sliders 57 and 58 so as to protrude the stopper 105 and the needle tubes 52 and 53 from the sheath 40. Then, the first regions R1 and the second regions R2 of the needle tubes 52 and 53 are restored by means of elasticity of super-elastic metal so as to be a shape before the needle tubes are received in the sheath as shown in FIG 21.

The user positions the target tissue T1 between the first needle tube 52 and the second needle tube 53 and retracts the sliders 57 and 58. Then, as shown in FIG 22, the needle tubes 52 and 53 are inserted into the target tissue T1, and the engagement protrusions 52A and 53A engage with each other in the inside of the target tissue T1, thereby forming the first needle tube 52 and the second needle tube 53 in a substantially annular shape.

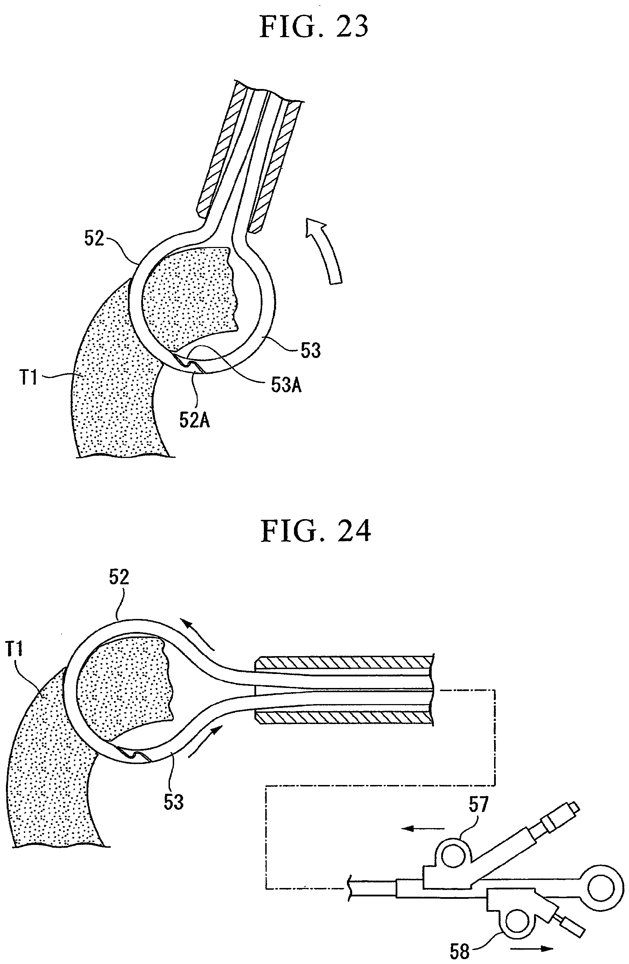

Subsequently, in order to release the anchors, the user moves the engagement portion between the needle tubes 52 and 53 to the outside of the target tissue T1. At this time, the user may rotate the annular needle tubes 52 and 53 by operating the endoscope device 100 as shown in FIG. 23, or may move the engagement portion between the needle tubes 52 and 53 by advancing one slider (for example, the first slider 57) and retracting the other slider (for example, the second slider 58) as shown in FIG 24.

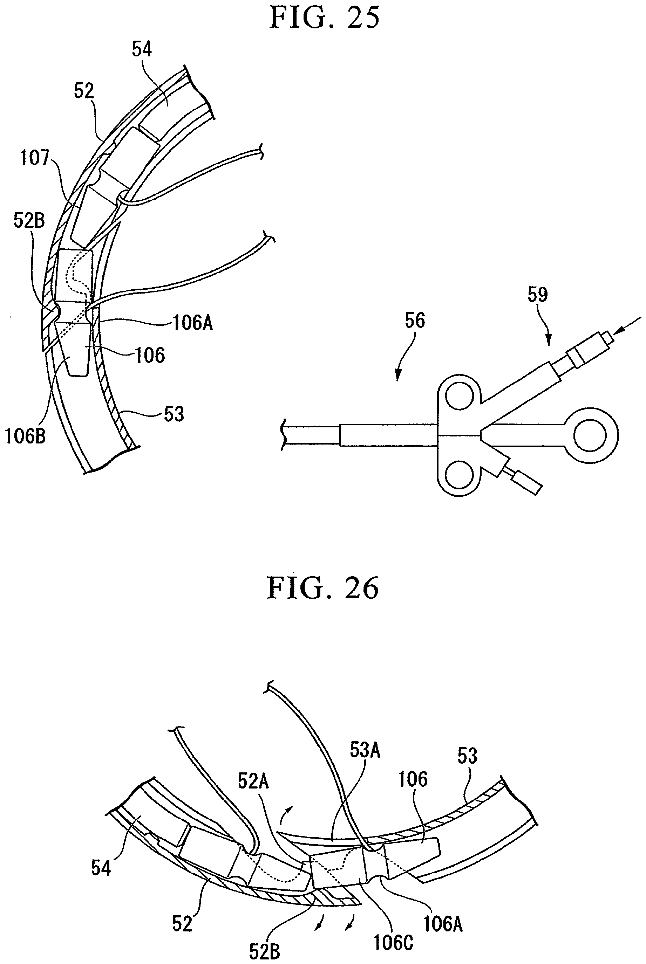

When the engagement portion moves to the outside of the target tissue T1, the user advances the first pusher 54 by operating the first pusher operation portion 59 as shown in FIG 25. The first anchor 106 and the second anchor 107 pushed by the first pusher 54 move in the inside of the first needle tube 52 toward the distal end so as to be close to the releasing protrusion 52B.

Since the angle of the inclined surface on the side of the proximal end portion of the releasing protrusion 52B is not more than the taper angle on the side of the distal end portion 106B of the first anchor 106, the distal end portion 106B hardly comes into contact with the releasing protrusion 52B, and the first anchor 106 smoothly moves toward the second needle tube 53.

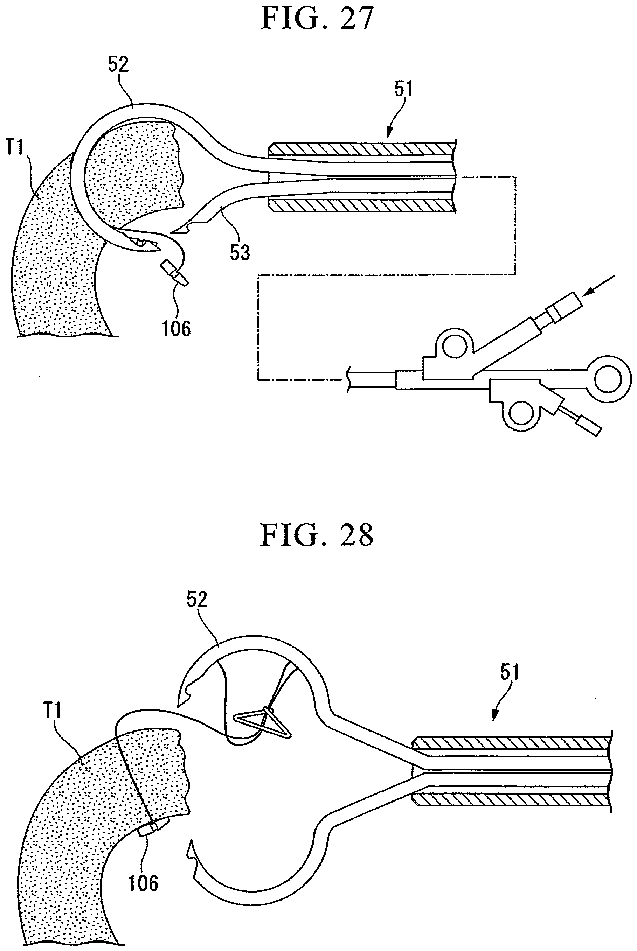

When the groove 106A moves to the second needle tube 53 rather than the releasing protrusion 52B, the proximal end portion 106C having a larger diameter comes into contact with the releasing protrusion 52B. As a result, as shown in FIG 26, the releasing protrusion 52B pushed by the proximal end portion 106C moves so as to be separated from the second needle tube 53, thereby releasing the engagement between the engagement protrusions 52A and 53A and releasing the first anchor 106 as shown in FIG 27. At this time, in the case where the first anchor 106 remains in the second needle tube 53, the first anchor 106 may be released by advancing the second pusher 55.

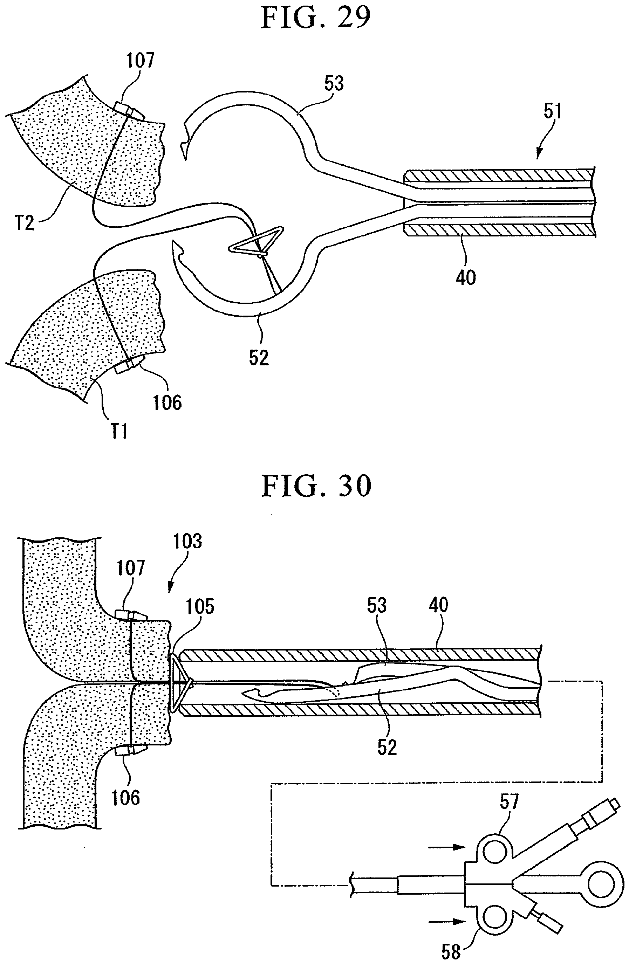

After the user takes out the first needle tube 52 from the target tissue T1 as shown in FIG 28, the user reverses the entire suturing device 51 around an axial direction and, as shown in FIG 29, locks the second anchor 107 on the target tissue T2 in the same sequence. Then, as shown in FIG 30, the user pulls the needle tubes 52 and 53 to the inside of the sheath 40 so as to move the stopper 105 toward the anchors 106 and 107, thereby suturing the treatment target portion. Finally, when the user sufficiently retracts the first pusher 54, the suturing unit 103 is separated from the suturing device 51, thereby ending the surgical technique.

In the suturing device 51 according to this embodiment, since the anchors 106 and 107 are released from the distal end of the first needle tube 52 curved in a circular-arc shape, it is possible to carry out the accurate surgical technique while appropriately observing the distal end of the needle tube and the anchor by means of the endoscope device 100.

When the internal configuration of the suturing device 51 according to this embodiment is modified, it is possible to further easily lock the anchor. Hereinafter, the modified example will be described.

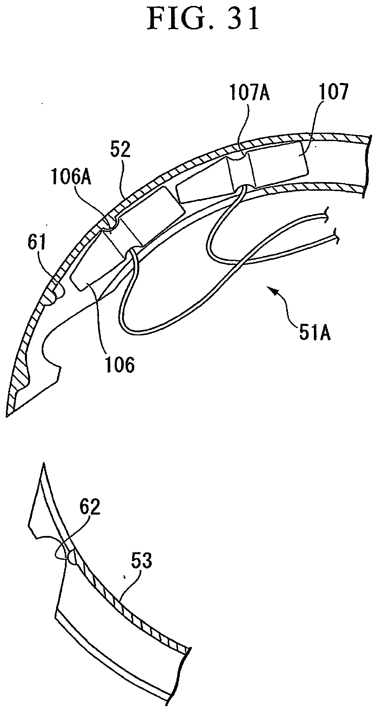

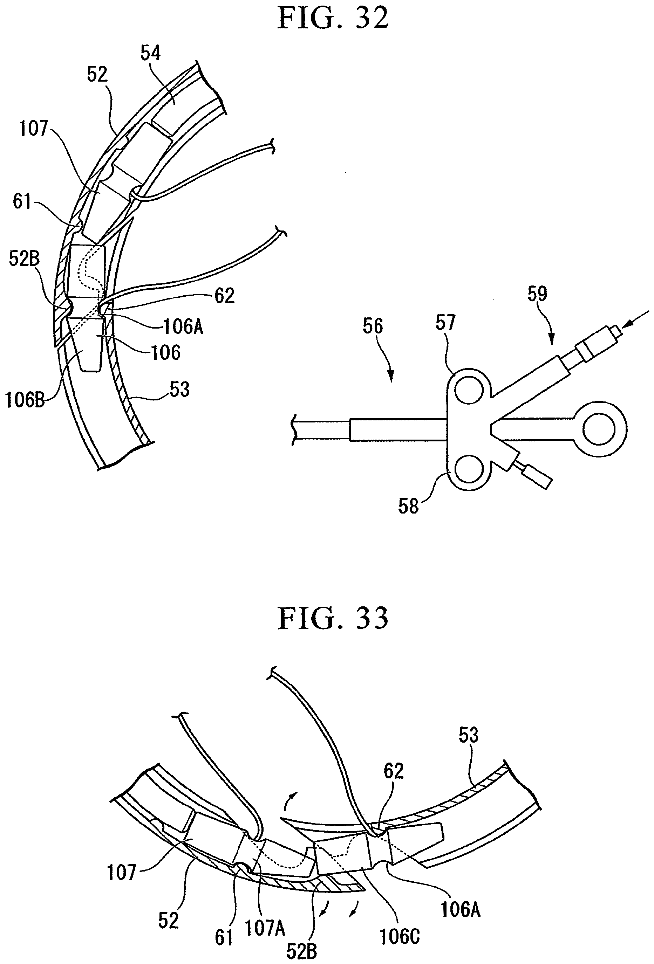

FIG 31 is an enlarged sectional view showing a portion in the vicinity of the distal ends of the needle tubes 52 and 53 of a suturing device 51A which is a modified example of the suturing device 51. Receiving protrusions 61 and 62 are circumferentially formed in the inner surfaces in the vicinity of the distal ends of the needle tubes 52 and 53, respectively, so as to deliver and receive the anchors. The receiving protrusions 61 and 62 can engage with the grooves 106A and 107A of the anchors 106 and 107, respectively. A length of each of the receiving protrusions 61 and 62 may be appropriately determined depending on strength for holding the anchors. Additionally, in the operation part 56, as shown in FIG 32, the first slider 57 is integrally formed with the second slider 58.

An operation upon using the suturing device 51A with the above-described configuration will be described.

In the same manner as the suturing device 51, the needle tubes 52 and 53 are inserted in the target tissue T1, and the slider is advanced so that the needle tubes 52 and 53 engage with each other in an annular shape in the inside of the target tissue T1. Subsequently, when the first pusher 54 is advanced, as shown in FIG 32, the groove 106A of the first anchor 106 engage with the receiving protrusion 62 of the second needle tube 53, and the first anchor 106 is delivered from the first needle tube 52 to be received in the second needle tube 53.

When the user further pushes the first pusher 54, as shown in FIG 33, the proximal end portion 106C of the first anchor passes over the releasing protrusion 52B, thereby releasing the engagement between the needle tubes 52 and 53. At the same time, the groove 107A of the second anchor 107 engages with the receiving protrusion 61 of the first needle tube 52.

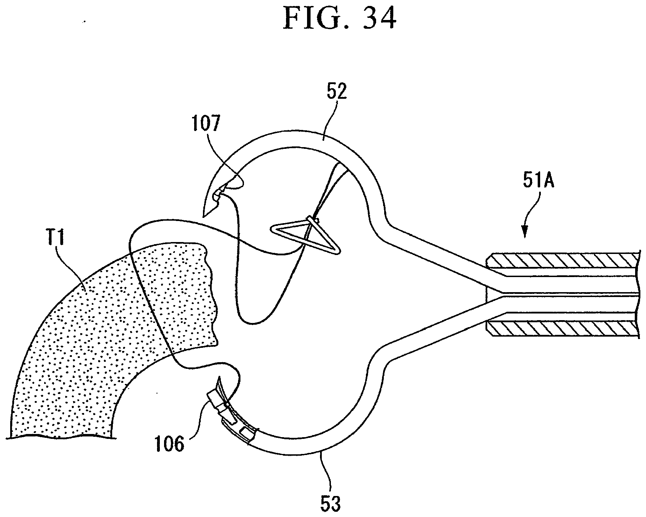

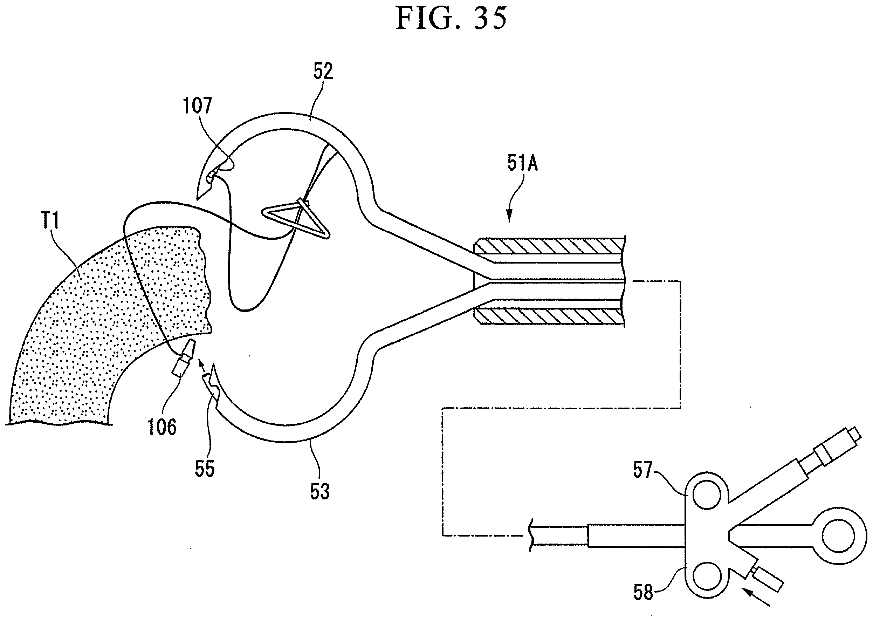

When the user retracts the sliders 57 and 58, as shown in FIG 34, the first needle tube 52 and the second needle tube 53 are taken out from the target tissue T1 in a state where the second anchor 107 and the first anchor 106 are respectively received in the vicinity of the distal ends of the needle tubes. Accordingly, the first anchor 106 which has been inserted from the first needle tube 52 into the target tissue T1 passes through the inside of the target tissue T1 and moves to the second needle tube 53. Then, when the user advances the second pusher 55 so as to push the first anchor 106 from the second needle tube 53, as shown in FIG 35, the releasing operation of the first anchor 106 ends.

The user reverses the suturing device 51A around an axial direction, and delivers the second anchor 107 to be received in the second needle tube 53 in the same sequence, thereby locking the second anchor on the target tissue T2.

In the suturing device 51A, the first anchor 106 received in the first needle tube 52 is delivered to be received in the second needle tube 53 in the inside of the target tissue T1 or the like. For this reason, it is possible that only the anchor penetrates the target tissue T1 or the like so as to move toward the second needle tube 53 for a locking operation thereon without the first needle tube 52 penetrating the target tissue T1 or the like. Accordingly, it is possible to further simply carry out the suturing operation.

Additionally, in the above-described suturing device 51A, a case is exemplified in which the anchor is delivered and received in a state where the first needle tube 52 and the second needle tube 53 engage with each other. However, the anchor can be delivered and received without engaging both needle tubes with each other so long as the distal ends of the needle tubes are ensured to satisfactorily face each other.

Further, in the suturing device 51A, since it is not necessary to expose the engagement portion between the needle tubes 52 and 53 to the outside of the target tissue T1 unlike the suturing device 51, as shown in FIGS. 32 and 35, no problem arises even when the first slider 57 is integrally formed with the second slider 58. Although it is possible to further simply carry out the operation in this case, it is not essential that the suturing device 51A have such configuration, and the sliders 57 and 58 may be configured to be independently slidable in the same manner as the suturing device 51.

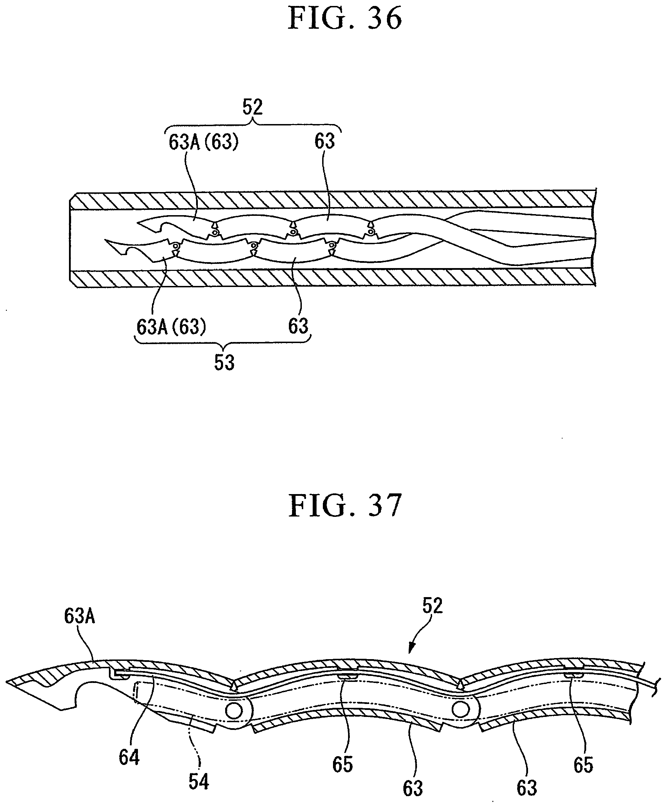

Furthermore, in the above-described suturing devices 51 and 51A, the structures of the needle tubes 52 and 53 are not limited to the above-described structures. For example, like a modified example shown in FIGS. 36 and 37, the needle tubes 52 and 53 may be configured such that plural needle members 63 are rotatably connected to each other.

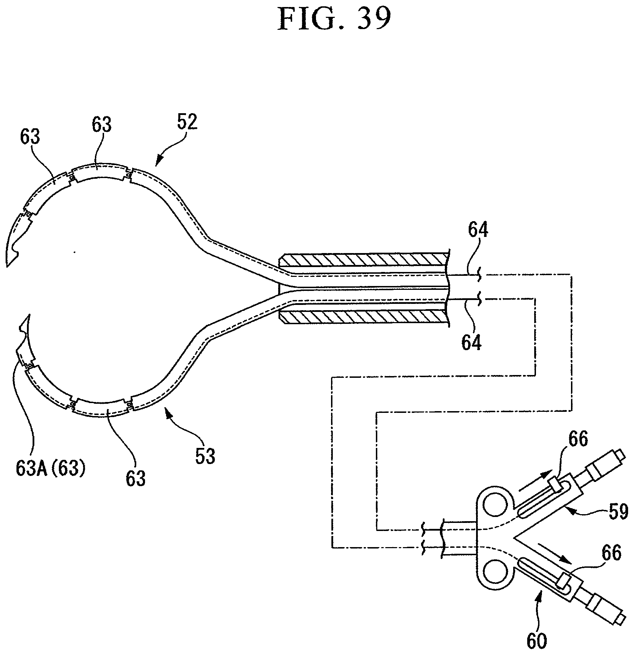

In this case, as shown in FIG 37, one end of a tension wire 64 for holding the needle members 63 in the shapes of the needle tubes 52 and 53 is fixed to a needle member 63A located on the side of the foremost distal end of each of the needle tubes 52 and 53 Then, the other end of the tension wire 64 extends up to each of the pusher operation portions 59 and 60 while passing through a guide 65 formed in the curved inner surface on the outer peripheral side of each needle member 63, and is mounted with an operation handle 66 (see FIG 39).

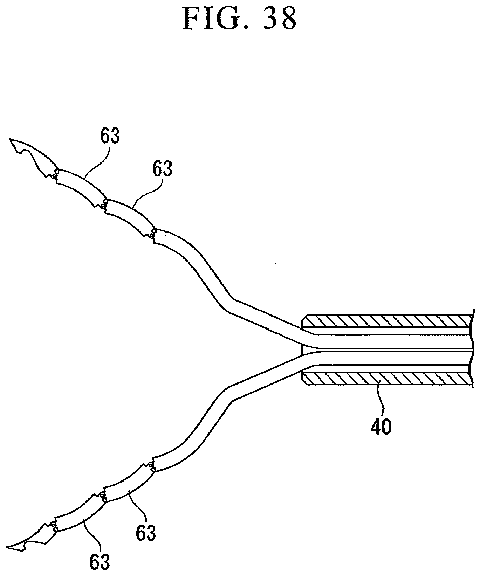

At the time of using the suturing device, after the sliders 57 and 58 are advanced so as to protrude the needle members 63 from the sheath 40 as shown in FIG 38, the tension wires 64 respectively connected to the needle tubes 52 and 53 are pulled toward the proximal ends by operating the handle 66. Then, as shown in FIG 39, the needle members 63 are held to be in the curved shape of the needle tubes 52 and 53, which can be inserted into the target tissue T1 or the like.

With such a configuration, when the tension wires 64 are pulled, the first regions R1 of the needle tubes 52 and 53 becomes substantially rigid, and when the tension wires 64 are loosened, the first regions R1 becomes substantially soft. As a result, it is possible to easily insert and extract the needle tubes 52 and 53 into or from the sheath 40 with a smaller force. Also, upon inserting the needle tube into the tissue, it is possible to further reliably carry out the puncture operation by allowing the needle tubes 52 and 53 to have sufficient rigidity.

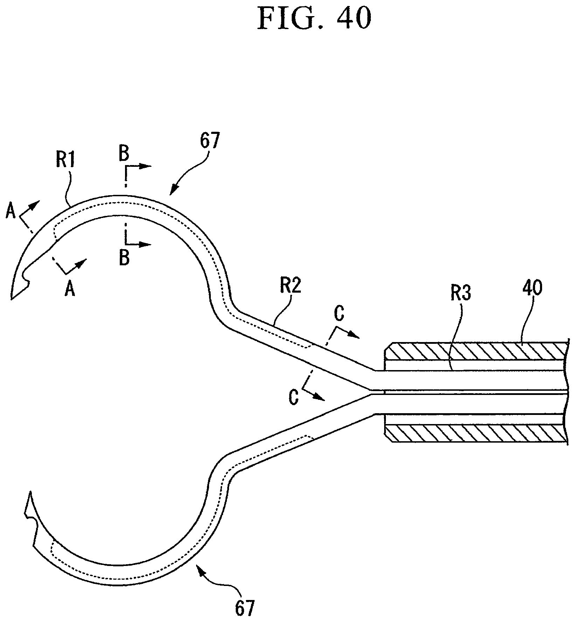

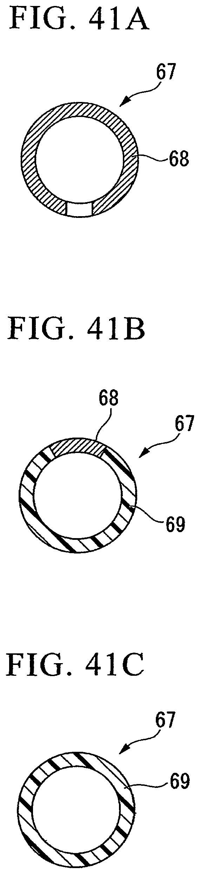

FIG 40 is a view showing a needle tube according to another modified example of the suturing device 51. FIGS. 41A, 41B, and 41C are sectional views respectively taken along the line A-A, the line B-B, and the line C-C. As shown in FIG. 41A, the distal end portion of the first region R1 of the needle tube 67 applied with a large force upon being inserted into the tissue is formed by only a rigid member 68 made of metal such as stainless steel. In the remainder portion of the first region R1 and the distal end portion of the second region R2 required to have constant elasticity, as shown in FIG 41B, a part, for example, only the outer peripheral portion of the curved shape thereof is formed by the rigid member 68, and the remainder portion is formed by a soft member 69 such as elastic resin. Then, the proximal end portion of the second region R2 and the third region R3 are formed by only the soft member 69. Likewise, the needle tube 67 is formed so that a ratio of the rigid member 68 gradually decreases in a direction toward the proximal end. As a result, even when super-elastic metal is not used as material, it is possible to form the needle tube 67 having desired elasticity so as to be received in the sheath 40 and to be formed in a desired curve shape at a time when the needle tube protrudes from the sheath 40. Such the needle tube 67 can be reliably formed by insert-molding or the like by means of the rigid member 68 formed in a shape shown in FIG 40. Note that, in the above description, a position where the ratio of the rigid material changes is an example, and may be appropriately changed in accordance with rigidity or elasticity required for the needle tube 67.

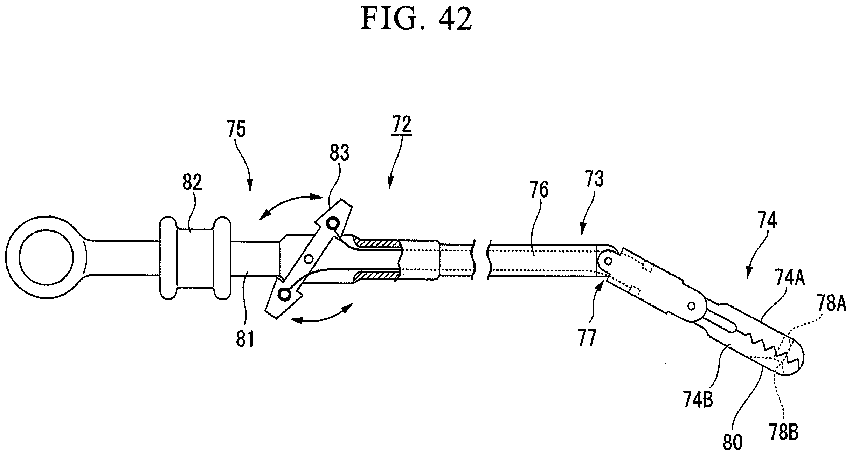

Next, a fourth embodiment will be described with reference to FIGS. 42 to 48. This embodiment provides a suturing system including an endoscope device, a suturing mechanism, and an endoscope forceps. In a suturing system 71 according to this embodiment, the endoscope device and the suturing mechanism may be appropriately configured as the endoscope device 100 and the suturing mechanism 33 described above.

FIG 42 is a view showing an example of the endoscope forceps 72 used in the suturing system 71. The endoscope forceps 72 include an insertion part 73 which is inserted into the body cavity, a gripping part 74 which is formed at the distal end of the insertion part 73, and an operation part 75 which is provided in the proximal end portion of the insertion part 73.

The insertion part 73 includes a soft portion 76 which has flexibility and a bending portion 77 which is rotatably supported by the distal end of the soft portion 76. A rotation surface of the bending portion 77 is aligned to be in parallel to a direction where the gripping part 74 is opened or closed. The bending portion 77 is connected to a transmission member such as a wire, and extends up to the operation part 75 via the insertion part 73.

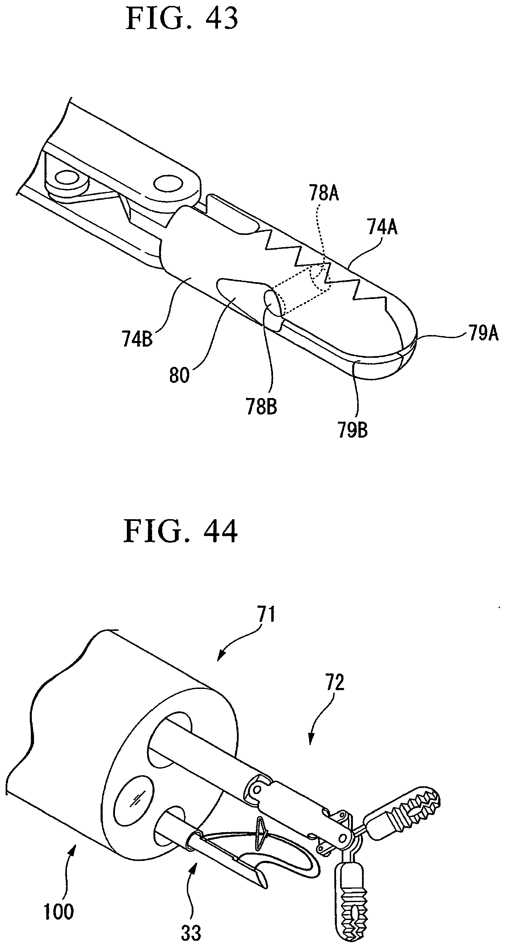

The gripping part 74 includes a pair of jaws 74A and 74B. The transmission member such as the wire used to open or close the jaws 74A and 74B extends up to the operation part 75. As shown in FIGS. 42 and 43, the jaws 74A and 74B are respectively provided with through-holes 78A and 78B which penetrate the jaws in the direction where the jaws 74A and 74B are opened or closed so as to communicate with each other, and slits 79A and 79B which respectively communicate with the through-holes 78A and 78B and extend up to the peripheral edges of the gripping surfaces of the jaws 74A and 74B facing each other. The inner diameters of the through-holes 78A and 78B are set to be smaller than the outer diameter of the sheath 40 of the suturing mechanism 33, thereby prohibiting the entry of the sheath 40.

Additionally, at least one of the jaws 74A and 74B is provided with a guide surface 80 which is formed in an inclined surface shape in which the opening peripheral edge of the through-hole located on the opposite side of the gripping surface becomes gradually deeper in a direction moving closer to the through-hole. The guide surface 80 may be provided in both jaws.

The operation part 75 includes a body 81 which is connected to the insertion part 73, a slider 82 which is used to operate the gripping part 74, and a bending operation portion 83 which is used to operate the bending portion 77. The slider 82 is connected to the transmission member extending from the gripping part 74. When the slider 82 is slid on the body 81, it is possible to open or close the gripping part 74. The bending operation portion 83 is configured as a bar-shaped member, and a center portion thereof in a longitudinal direction is rotatably supported by the body 81. Both longitudinal end portions of the bending operation portion 83 are connected to the transmission members extending from the bending portion 77. When the bending operation portion 83 is rotated, as shown in the drawing, it is possible to change a direction of the gripping part 74 by changing an angle formed between the bending portion 77 and the soft portion 76.

An operation upon using the suturing system 71 with the above-described configuration will be described. Upon using the suturing system 71, the suturing mechanism 33 and the endoscope forceps 72 are inserted into the channel of the endoscope device 100 so as to protrude therefrom as shown in FIG 44. The timing when the suturing mechanism 33 and the endoscope forceps 72 are inserted may be the timing before or after the endoscope device 100 is inserted into the body cavity.

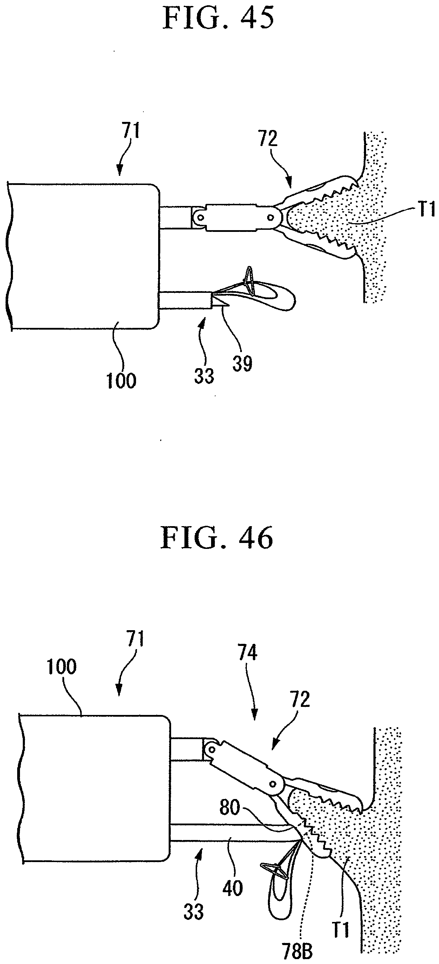

A user introduces the endoscope device 100 into a position in the vicinity of the treatment target portion, and grips the target tissue T1 by means of the endoscope forceps 72 as shown in FIG 45. At this time, the guide surface 80 is disposed so as to face the suturing mechanism 33.

The user operates the bending operation portion 83 while gripping the target tissue T1 so as to allow the gripping part 74 to face the suturing mechanism 33. At this time, it is desirable that the needle tube 39 of the suturing mechanism 33 is received in the sheath 40 so as not to be erroneously inserted into the target tissue T1.

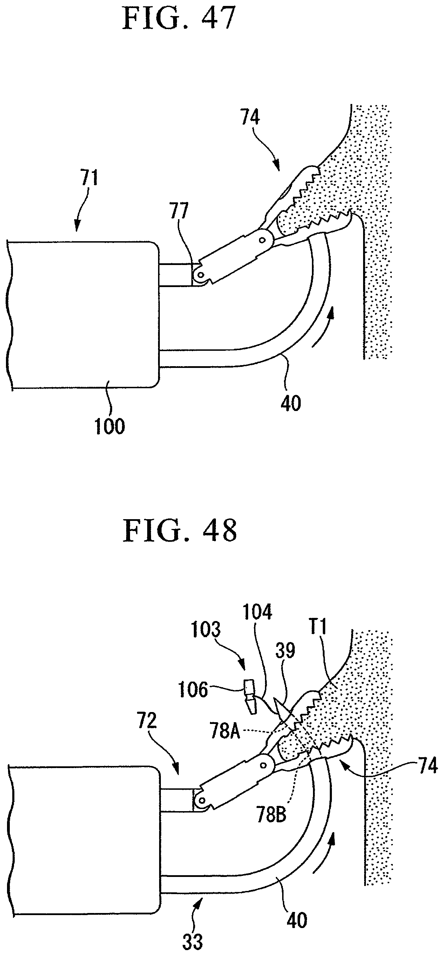

When the gripping part 74 faces the suturing mechanism 33, the guide surface 80 moves to a position in front of the suturing mechanism 33. When the user advances the sheath 40, the distal end of the sheath 40 is guided by the guide surface 80 so as to be close to the through-hole 78B, and the inner cavity of the sheath 40 communicates with the through-hole 78B. When the user further pushes the sheath 40 in a forward direction, the distal end of the sheath 40 rotates together with the gripping part 74 about the proximal end of the bending portion 77, and the distal end portion of the sheath 40 is curved so as to be substantially perpendicular to the axis of the endoscope device 100 as shown in FIG 47.

When the user advances the slider 19 in this state, the needle tube 39 penetrates the target tissue T1 via the through-hole 78B as shown in FIG 48, and passes through the through-hole 78A to protrude therefrom. In this manner, it is possible to release the first anchor 106 to be locked on the target tissue T1. When the gripping state of the gripping part 74 is released after the anchor is locked thereon, the suturing thread 104 of the suturing unit 103 is separated from the gripping part 74 via the slits 79A and 79B.

In the suturing system 71 according to this embodiment, by gripping the target tissue with the endoscope forceps 72 having the bending portion 77, the sheath 40 of the suturing mechanism 33 rotates together with the gripping part 74 so that the distal end portion is curved so as to be substantially perpendicular to the axis of the endoscope device 100. As a result, since it is possible to release the anchor while reliably observing the distal end of the protruding needle tube 39 by means of the endoscope device 100, it is possible to improve reliability of the anchor releasing operation.

Further, since the guide surface 80 is provided in the through-hole, even when a position of the through-hole slightly deviates from the axis of the suturing mechanism 33, the distal end of the sheath 40 is reliably guided so that the inner cavity of the sheath 40 communicates with the through-hole.

Furthermore, since the diameter of the through-hole is set to be smaller than the outer diameter of the sheath 40, it is possible to prevent a problem that the sheath 40 enters the through-hole so that the sheath is caught therein and is not separated therefrom. Accordingly, it is possible to further easily carry out the surgical technique.

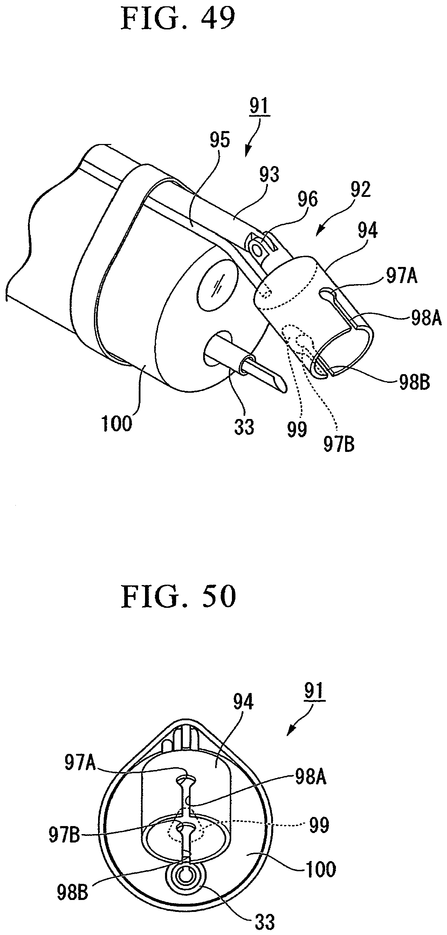

Next, a fifth embodiment will be described with reference to FIGS. 49 to 54. A suturing system 91 according to this embodiment is different from the suturing system 71 according to the fourth embodiment in that a suction cap is used instead of the endoscope forceps.

FIG 49 is a view showing a distal end portion of the suturing system 91 at the time of using. In the suturing system 91, a suction cap 92 tied with the endoscope device 100 and inserted into the body cavity is used instead of the endoscope forceps 72.

The suction cap 92 includes an insertion part 93 which is inserted into the body cavity, a cap part 94 which is mounted to the distal end of the insertion part 93, and a suction tube 95 which is connected to the cap part 94.

The insertion part 93 includes a bending portion 96, and the cap part 94 can be operated so as to be curved toward the suturing mechanism 33 as shown in FIG 49.

As shown in FIGS. 49 and 50, the cap part 94 is configured as a cylindrical member having a bottom, where through-holes 97A and 97B and thread-extraction slits 98A and 98B are formed at the same position as those of the gripping part 74 of the above-described endoscope forceps 72. In the same manner as the through-hole 78B, a guide surface 99 is formed in the through-hole 97B in the side of the suturing mechanism 33.

The distal end of the suction tube 95 is connected to a bottom surface of the cap part 94 so as to be opened to the inside of the cap part 94, and the proximal end thereof is connected to a suction mechanism (not shown). Then, when the suction mechanism is operated, a negative pressure occurs in the inside of the cap part 94 so as to suck the tissue located inside the cap part 94.

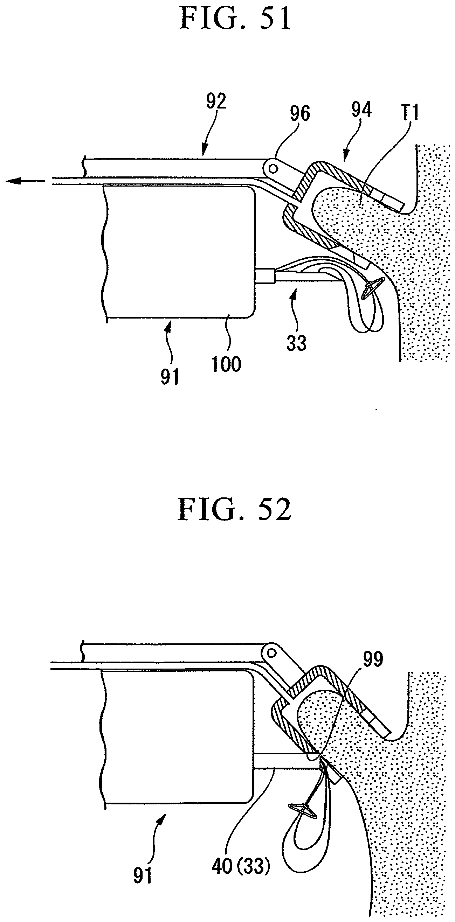

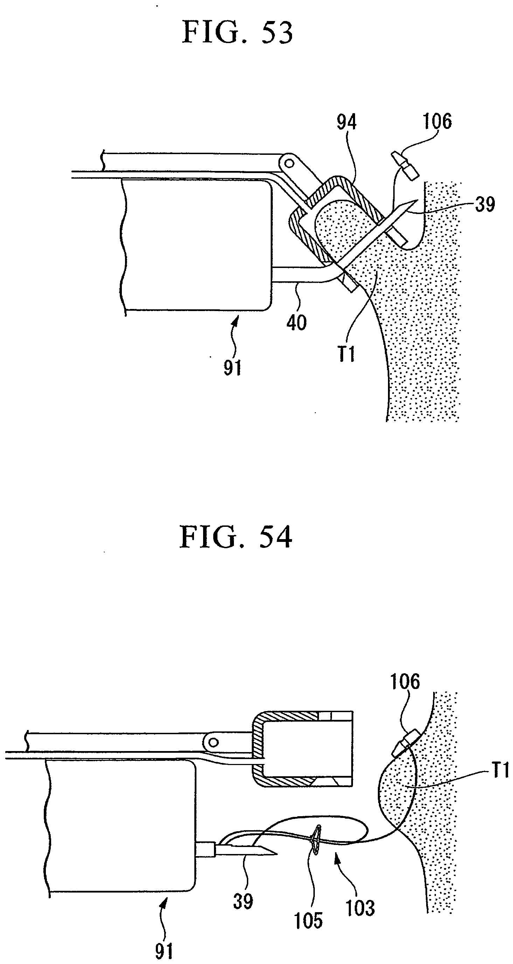

The suturing system 91 with the above-described configuration is capable of carrying out the releasing operation and the locking operation of the anchors in the substantially same sequence as that of the suturing system 71. That is, as shown in FIG 51, the bending portion 96 is operated so as to be curved toward the suturing mechanism 33 in a state where the target tissue T1 is sucked by the cap part 94. Then, the distal end of the sheath 40 comes into contact with the guide surface 99 as shown in FIG. 52, and then the needle tube 39 passes through the target tissue T1 to release the first anchor 106 as shown in FIG 53, thereby locking the first anchor 106 on the target tissue T1 as shown in FIG 54.

Even in the suturing system 91 according to this embodiment, it is possible to reliably release the anchors 106 and 107 while observing the distal end of the needle tube 39 penetrating the target tissue T1 by means of the endoscope device 100 in the same manner as the suturing system 71.

Note that, in the above-described fourth and fifth embodiments, a case is exemplified in which the suturing mechanism is inserted into the channel of the endoscope device. However, instead, the suturing mechanism may be attached to the outside of the endoscope device so as to be relatively movable with respect to the endoscope device in a longitudinal direction.

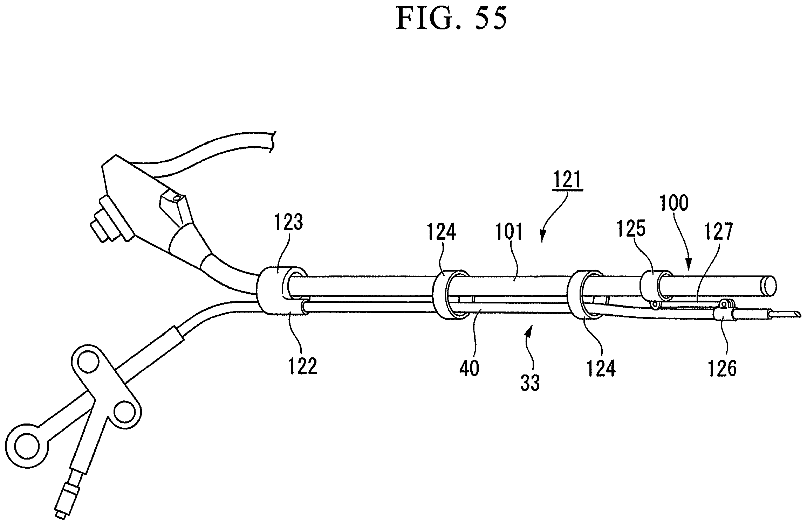

Next, a sixth embodiment will be described with reference to FIGS. 55 to 58. A suturing system 121 according to this embodiment is different from the suturing systems according to the above-described embodiments in that the distal end portion of the endoscope device as an observation member is movable up to a position where the needle tube of the suturing mechanism after penetrating the target tissue can be observed.

FIG 55 is an overall view showing the suturing system 121. Although the suturing system 121 includes the endoscope device 100 and the suturing mechanism 33, the suturing mechanism 33 is not inserted into the channel of the endoscope device 100, but is tied with the endoscope device 100 in a state where both insertion parts thereof are disposed in parallel to each other.

As shown in FIG 55, the suturing mechanism 33 is inserted through a scope holder 122, and the endoscope device 100 is inserted through a first guide 123 mounted to the scope holder 122 in a freely advancing and retracting manner. Then, an insertion part 101 of the endoscope device 100 is tied with the sheath 40 of the suturing mechanism 33 by means of auxiliary guides 124. The number of the auxiliary guides 124 may be appropriately determined in accordance with a length of the insertion part 101 or the sheath 40. Additionally, the auxiliary guide 124 may be fixed to any one of the insertion part 101 and the sheath 40.

A scope fixing band 125 is mounted to the distal end portion of the insertion part 101, and a suturing mechanism fixing band 126 is mounted to a position located at the distal end portion of the suturing mechanism 33 and located closer to the distal end than the scope fixing band 125. The scope fixing band 125 is connected to the suturing mechanism fixing band 126 by means of a rigid link 127. Both ends of the link 127 are pivotally supported by the scope fixing band 125 and the suturing mechanism fixing band 126, respectively, in a freely rotating manner. The both ends of the link 127 are pivotally supported so as to rotate on the same plane.

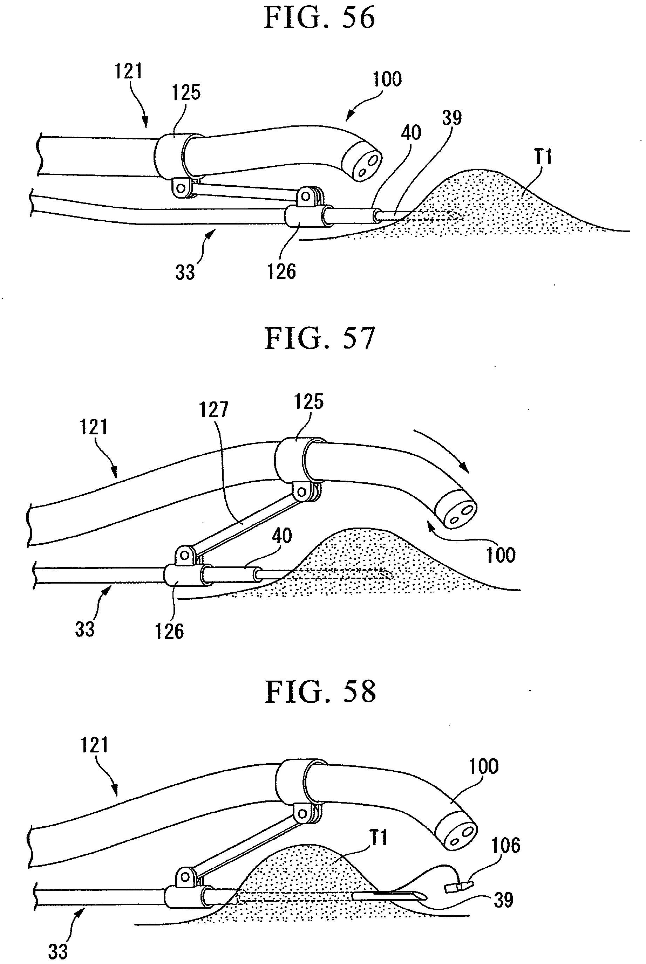

An operation upon using the suturing system 121 with the above-described configuration will be described. First, the suturing system 121 is introduced into the body cavity, and the needle tube 39 is inserted into the target tissue T1. At this time, as shown in FIG 56, since the distal end of the endoscope device 100 is located in the vicinity of the distal end of the sheath 40 in an initial state where the suturing mechanism fixing band 126 is located in front of (at a position closer to the distal end than) the scope fixing band 125, it is possible to reliably observe the insertion operation of the needle tube 39.

In a state where the needle tube 39 is located in the inside of the target tissue T1, the user pushes forward the endoscope device 100 while holding the scope holder 122. Then, as shown in FIG 57, the link 127 rotates about the end portion on the side of the suturing mechanism fixing band 126 such that the distal end of the treatment endoscope device 100, to which the scope fixing band 125 is fixed, moves to a position in front of the distal end of the sheath 40.

When the needle tube 39 penetrates the target tissue T1 in this state, since it is possible to reliably observe the distal end of the needle tube 39 by means of the treatment endoscope device 100 as shown in FIG 58, it is possible to reliably release the first anchor 106 or the like.

In the suturing system 121 according to this embodiment, when the sheath 40 of the suturing mechanism 33 is formed to have rigidity in which the sheath is not easily bent by the pushing operation and the rotation operation of the above-described treatment endoscope device 100, it is desirable in that the distal end of the endoscope device 100 smoothly moves. Additionally, from the view point of reliably maintaining the insertion performance of the tied endoscope device into the body cavity, it is desirable that the endoscope device 100 is configured as a so-called thin scope in which the diameter of the insertion part 101 is about 5 to 8 mm.

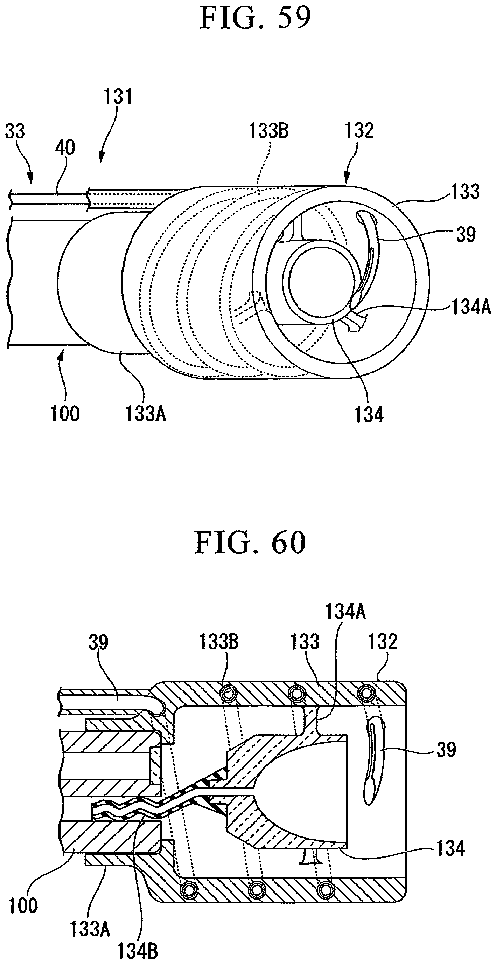

Next, a seventh embodiment will be described with reference to FIGS. 59 to 64. A suturing system 131 according to this embodiment is different from the suturing systems according to the above-described embodiments in that the needle tube of the suturing mechanism protrudes in a different manner.

FIG 59 is a perspective view showing the distal end portion of the suturing system 131. The distal end of the sheath 40 of the suturing mechanism 33 is connected to a puncture cap 132 used for a mounting operation to the distal end of the endoscope device 100.

FIG 60 is a sectional view showing the puncture cap 132 along an axial direction. The puncture cap 132 includes an outer cylindrical body 133 and a suction cup 134 which is mounted to the inside of the cylindrical body 133.

The cylindrical body 133 is configured as a substantially cylindrical member made of a resin or the like. When a fitting portion 133A located on the side of the proximal end is fitted to the distal end of the treatment endoscope device 100, both portions can be connected to each other. Additionally, a wall surface of the cylindrical body 133 is provided with a spiral needle tube channel 133B, through which the needle tube 39 is inserted. The distal end of the needle tube 133B is opened to the inner surface of the cylindrical body 133.

The needle tube 39 according to this embodiment is made of super-elastic metal in the same manner as the above-described needle tube, and is curved in a spiral loop shape so as to be smoothly inserted through the needle tube channel 133B. However, a region with a predetermined length, which protrudes in use from the distal end of the needle tube channel 133B, is more curved than other regions of the loop.

The suction cup 134 is supported by an inner wall of the cylindrical body 133 via a support portion 134A. When a proximal end 134B of the suction cup 134 is inserted into the channel of the endoscope device 100, the proximal end air-tightly comes into contact with the channel. Accordingly, when the suction cup 134 is connected to the channel, and the channel is connected to a suction device (not shown), it is possible to suck the tissue by means of the suction cup 134.

It is desirable that the cylindrical body 133 and the suction cup 134 be made of a transparent resin or the like in order to reliably observe the tissue sucked by the suction cup 134 by means of the endoscope device. For example, polychlorinated biphenyl (PCB) having high transparency may be appropriately adopted.

An operation upon using the suturing system 131 with the above-described configuration will be described. A user first mounts the cylindrical body 133 to the distal end of the endoscope device 100 so that the proximal end 134B of the suction cup 134 is connected to the channel of the endoscope device 100, and inserts the suturing system 131 into the body cavity so that the distal end thereof moves to a position in the vicinity of the target tissue T1.

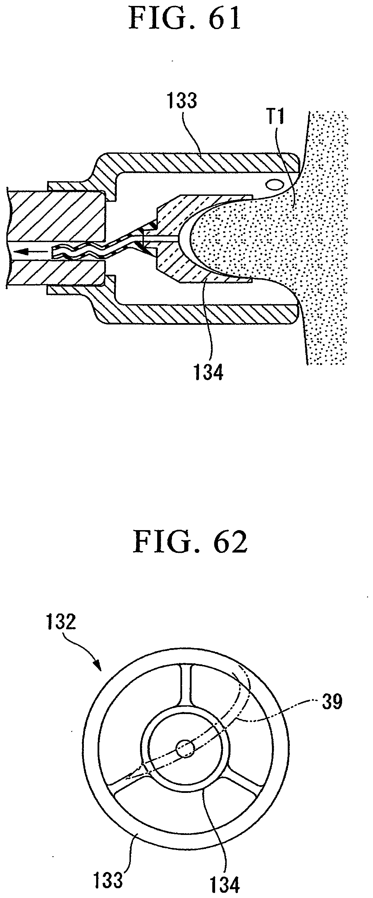

Next, the user pushes the cylindrical body 133 against the target tissue T1 and operates the suction device, thereby sucking a part of the target tissue T1 into the inside of the cylindrical body 133 by means of the suction cup 134 as shown in FIG. 61. Then, the user operates the slider 19 so as to advance the needle tube 39.

The needle tube 39 advances in the inside of the needle tube channel 133B of the cylindrical body 133, and protrudes from an opening located in the distal end of the needle tube channel 133B to the inside of the cylindrical body 133. As described above, since the distal end portion of the needle tube 39 is more curved than other regions, as shown in FIG 62, the distal end portion protrudes toward the axis of the puncture cap 132. That is, the needle tube 39 protrudes in a direction where the needle tube is easily inserted into the part of the target tissue T1 sucked by the suction cup 134.

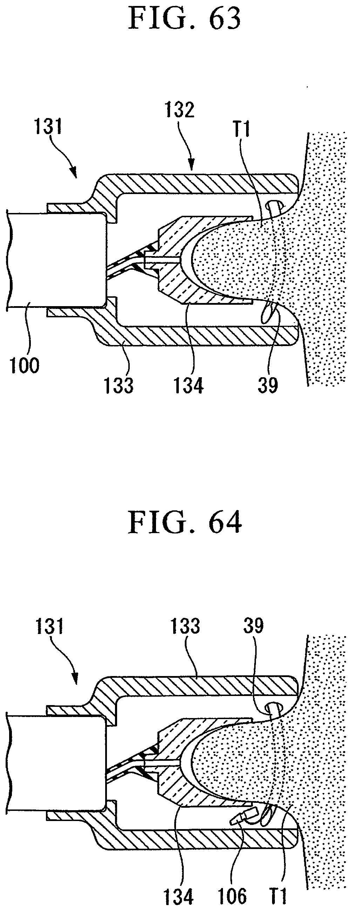

Accordingly, when the user further advances the needle tube 39 in a forward direction, as shown in FIG 63, the needle tube 39 is inserted into the target tissue T1 so as to penetrate the tissue. Since the needle tube 39 is inserted into the target tissue T1 so as to penetrate the tissue in the inside of the cylindrical body 133, the user is capable of visibly recognizing a series of movements of the distal end of the needle tube 39 in a reliable manner by means of the endoscope device 100.

Subsequently, as shown in FIG 64, the first anchor 106 is released from the needle tube 39 in the substantially same sequence as those of the above-described embodiments. When the needle tube 39 is taken out from the target tissue T1 and the suction operation of the suction cup 134 is stopped, the operation of locking the first anchor 106 on the target tissue T1 ends.

In the suturing system 131 according to this embodiment, the user is capable of reliably releasing the anchor just by sucking the target tissue T1 by means of the suction cup 134 and operating the needle tube 39 by means of the slider 19. Accordingly, since it is not necessary to carry out the second-stage operation in which the target tissue is gripped and then curved toward the suturing mechanism 33 unlike the suturing systems 91 and 121, it is possible to further simply and reliably carry out the suturing operation including the releasing operation of the anchors.

In the above-described embodiments, a case is exemplified in which the target tissue T1 is sucked and held by the suction cup 134, but the target tissue may be held by other means. For example, the tissue may be directly sucked by the cylindrical body 133 or the target tissue may be gripped by the forceps or the like protruding from the channel of the endoscope device 100.

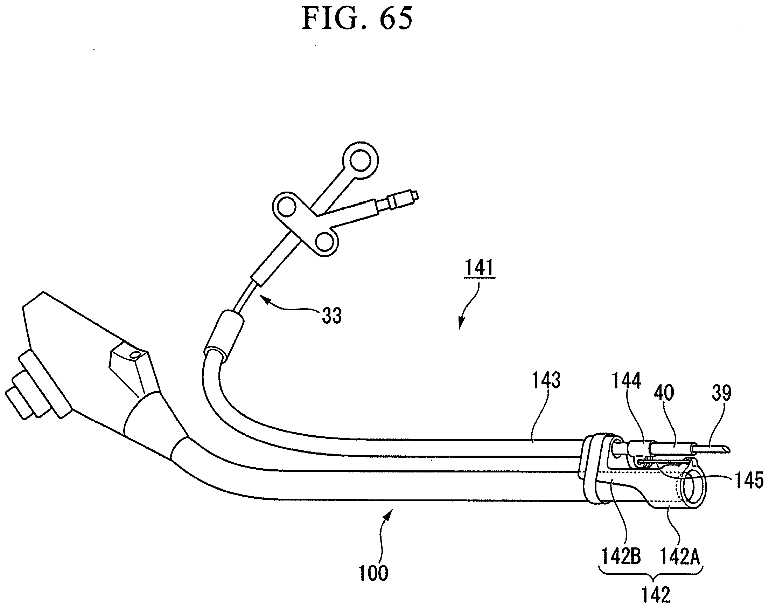

Next, an eighth embodiment will be described with reference to FIGS. 65 to 68. A suturing system 141 according to this embodiment is different from the suturing systems according to the above-described embodiments in that the suturing mechanism is connected to the endoscope device in a different manner.

FIG 65 is an overall view showing a configuration of the suturing system 141. The suturing mechanism 33 is inserted through a cap sheath 143 of which a distal end is mounted with a lifting cap 142. The lifting cap 142 includes a cylindrical body 142A located on the side of the distal end and a sleeve 142B located on the side of a proximal end.

Similar to the puncture cap 132, the cylindrical body 142A can be fixed to the endoscope device 100 by fitting the distal end portion of the endoscope device 100 to the cylindrical body. The sleeve 142B is made of a soft material, and supports the cap sheath 143 so that the distal end is not separated from the endoscope device 100.

A link fixing portion 144 is fixed to the distal end of the sheath 40 protruding from the cap sheath 143. The link fixing portion 144 is connected to an outer peripheral surface of the cylindrical body 142A via a rigid link 145 which is substantially equal to the link 127 of the suturing system 121. The link 145 is movable with respect to the link fixing portion 144 and the cylindrical body 142A.

An operation upon using the suturing system 141 with the above-described configuration will be described. A user first mounts the lifting cap 142 to the distal end of the endoscope device 100, and inserts the suturing mechanism 33 into the cap sheath 143 form the proximal end. The insertion operation of the suturing mechanism 33 may be carried out after the endoscope device 100 and the cap sheath 143 are inserted into the body cavity.

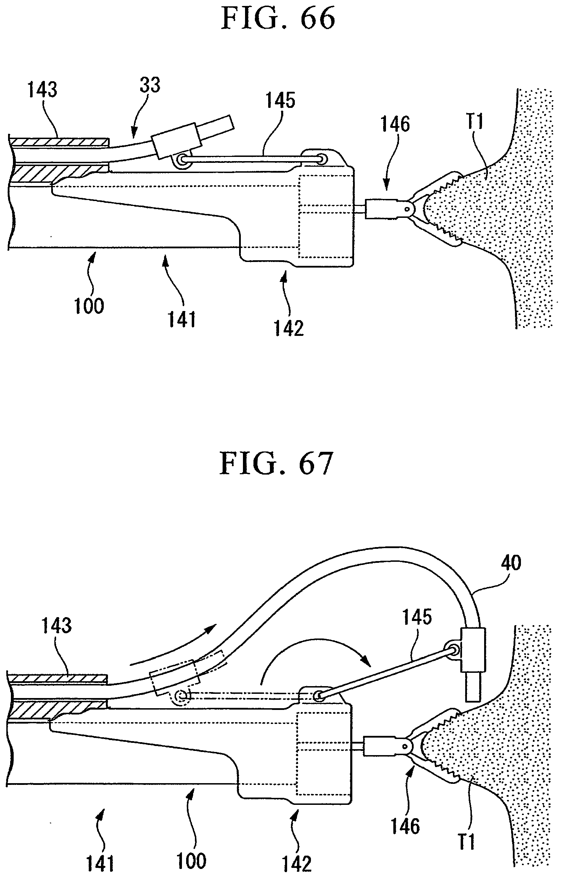

Next, as shown in FIG 66, the user grips the target tissue T1 by means of an endoscope forceps 146 inserted through the endoscope device 100. Since the endoscope forceps 146 does not include a portion such as the bending portion 77 of the endoscope forceps 72, the endoscope forceps only moves straight to grip the target tissue T1.

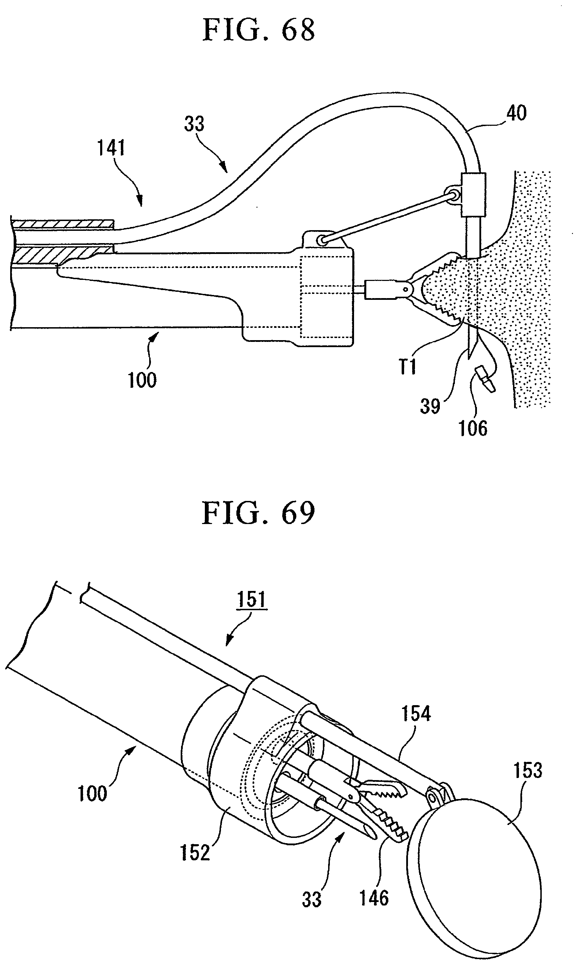

In a state where the target tissue T1 is gripped by the endoscope forceps 146, the user pushes the suturing mechanism 33 toward the distal end while holding the cap sheath 143, and advances the suturing mechanism 33 with respect to the cap sheath 143. When the suturing mechanism 33 is further advanced, the link 145 rotates about an end portion thereof on the side of the lifting cap 142 such that, as shown in FIG 67, the distal end of the sheath 40 moves to a position in front of the endoscope device 100, and the opening on the side of the distal end faces the target tissue T1 gripped by the forceps 146. In this state, as shown in FIG 68, the user operates the slider 19 so that the needle tube 39 penetrates the target tissue T1 and then the first anchor 106 is released therefrom.

In the suturing system 141 according to this embodiment, when the suturing mechanism 33 is advanced, the distal end of the sheath 40 moves to a position in front of the endoscope device 100 by means of the link 145, and so the needle tube 39 protrudes so as to be substantially perpendicular to the axis of the endoscope device 100. Accordingly, it is possible to carry out the releasing operation of the anchor while reliably observing the distal end of the needle tube 39 after penetrating the target tissue T1 by means of the endoscope device 100.

Next, a ninth embodiment will be described with reference to FIGS. 69 to 72. A suturing system 151 according to this embodiment is different from the suturing systems according to the above-described embodiments in that a second observation member is provided in addition to the endoscope device.

FIG 69 is a view showing a distal end portion of the suturing system 151. The forceps 146 and the suturing mechanism 33 are inserted through the channels of the endoscope device 100. A shaft member 154, of which a distal end is mounted with a mirror 153 as a second observation member, is inserted through a cap 152 mounted to the distal end portion of the endoscope device in a freely advancing and retracting manner. The mirror 153 is mounted with an operation member such as a wire (not shown). When the operation member is operated, it is possible to adjust an angle formed between the mirror 153 and the shaft member 154.

In the suturing system 151 with the above-described configuration, as shown in FIG 70, it is possible to observe the distal end of the needle tube 39 penetrating the target tissue T1 by seeing an image of the distal end reflected by the mirror 153 by means of the endoscope device 100. Accordingly, it is possible to reliably release the anchors while visibly recognizing the distal end of the needle tube 39. At this time, an angle formed between the mirror 153 and the shaft member 154 may be appropriately adjusted so as to obtain a satisfactory field of view.

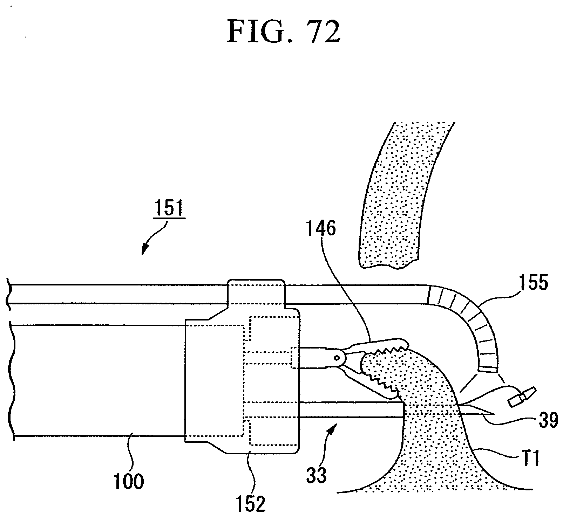

Additionally, the second observation member is not limited to the mirror 153, but may be, for example, a thin endoscope 155 shown in FIG 71. Even in this case, as shown in FIG 72, it is possible to further reliably release the anchors while observing the distal end of the needle tube 39 penetrating the target tissue T1 by means of the thin endoscope 155.

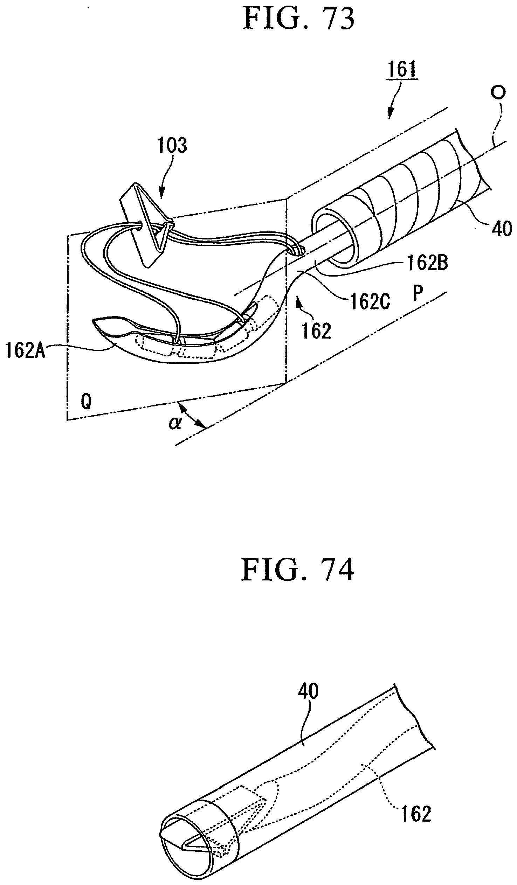

Next, a tenth embodiment will be described with reference to FIGS. 73 to 78. A suturing device 161 according to this embodiment is different from the suturing devices or the suturing systems according to the above-described embodiments in that the needle tube protruding from the sheath can be changed to a puncture shape described below.

FIG 73 is a view showing a distal end portion of the suturing device 161. A needle tube 162 has a distal end portion 162A which receives the suturing unit 103 and is formed in a substantially circular-arc shape. Further, at a boundary 162C between the distal end portion 162A and a proximal end portion 162B formed in a substantially linear shape, the distal end portion 162A is inclined so as to form an angle α with respect to the proximal end portion 162B.

As shown in FIG 73, the angle α is an angle formed by a plane Q including the curved distal end portion 162A and a plane P including an axis O of the proximal end portion 162B, and is set to be larger than 0 in general, where an upper limit is set to 90°. In a state where the needle tube 162 protrudes from the sheath 40, as shown in FIG 73, the needle tube 162 is naturally deformed to a shape (hereinafter, referred to as "puncture shape") suitable for the insertion operation into the tissue.

Further, when the needle tube 162 is received in the sheath 40, as shown in FIG 74, the needle tube 162 is deformed in a substantially linear shape in a degree that the sheath 40 basically formed in a substantially linear shape is not deformed. Also, flexibility of the needle tube 162 is maintained in a degree that the sheath 40 receiving the needle tube 162 can be inserted into the channel of the endoscope device 100. In order that the needle tube 162 has such characteristics, the distal end portion 162A of the needle tube 162 according to this embodiment is made of super-elastic alloy.

Furthermore, at the time when the needle tube 162 punctures the target tissue or the like as described below, since the whole part of the needle tube 162 rotates about a center axis of the proximal end portion 162B, the proximal end portion 162B is made of a material having torsion rigidity enough for a transmission of a rotary torque input from the operation part.

As a specific example, the proximal end portion 162B may be configured as a super-elastic alloy pipe or the proximal end portion 162B may be configured as a tubular member formed by a closely wound coil. It is possible to effectively improve torsion rigidity if the closely wound coil is formed by multiple thread winding method of winding multiple wires arranged in parallel or multilayer winding method of coaxially arranging multiple coils.

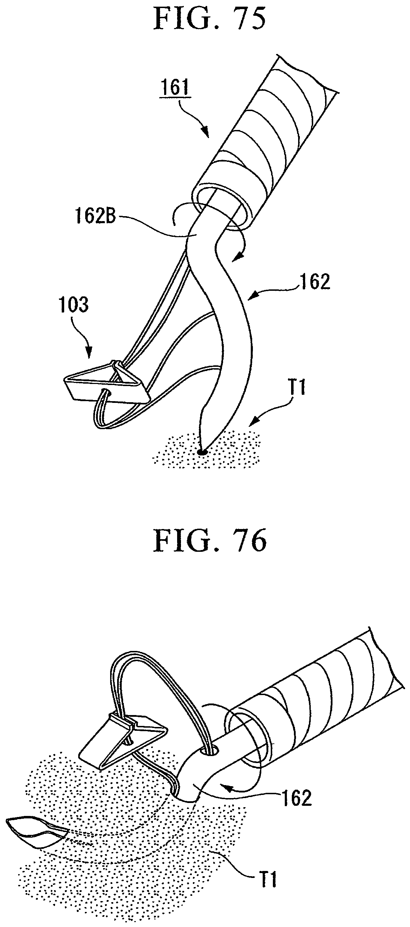

At the time when the needle tube 162 punctures the tissue, the user operates an operation part (not shown) so that the needle tube 162 rotates about the center axis of the proximal end portion 162B and a distal end of the needle tube comes into contact with the target tissue T1 as shown in FIG 75. Subsequently, as shown in FIG 76, the needle tube 162 is rotated to be inserted into or separated from the target tissue T1 in the same manner as a general surgical operation in which the suturing operation is carried out by holding a curved needle by a needle forceps. Since the angle α is set to be more than 0° and equal to or less than 90°, it is possible to insert and separate the needle tube 162 into or from the target tissue just by rotating the needle tube 162 in an axial direction. Additionally, since the distal end portion 162A of the needle tube 162 is inclined with respect to the center axis O of the proximal end portion 162B, it is possible to insert or separate the needle tube 162 under the observation using the endoscope device 100. Accordingly, in the same manner as the suturing devices and the suturing systems according to the above-described embodiments, it is possible to reliably carry out the suturing operation.

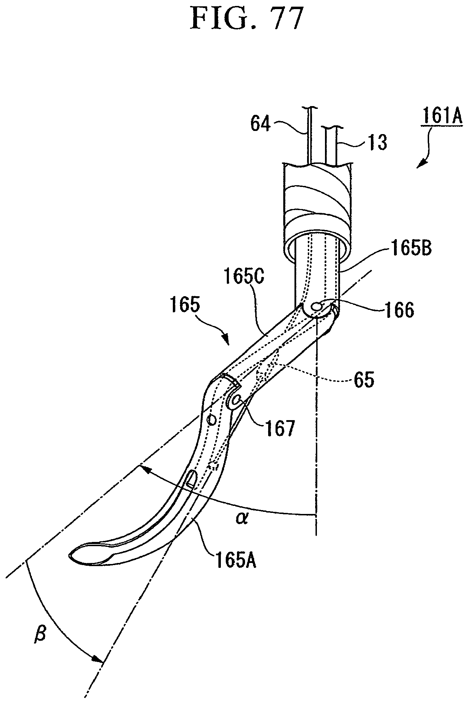

FIG 77 is a view showing a suturing device 161A according to a modified example of this embodiment. The received suturing unit 103 is omitted in the drawing. A needle tube 165 includes a distal end portion 165A, a proximal end portion 165B, and a middle portion 165C between the distal end portion 165A and the proximal end portion 165B, and the respective portions are rotatably connected to each other by means of a first rotary pin 166 and a second rotary pin 167. The middle portion 165C can rotate about the first rotary pin 166 in the range that an angle formed between the proximal end portion 165B and the middle portion 165C is within the above-described angle α. In the same manner, the distal end portion 165A can rotate about the second rotary pin 167 so that an angle formed between an axis of the side of the proximal end of the front end portion 165A and the middle portion 165C is within an angle β. An upper limit of the angle β may be set to 90° in the same manner as the angle α.

In the same manner as the modified example shown in FIGS. 36 to 39, the tension wire 64 is inserted through the inner cavity of the needle tube 165 and the pusher 13 is also inserted therethrough. The distal end of the tension wire 64 is fixed to an inner wall of the distal end portion 165A. In the same manner as the modified example of the third embodiment, the tension wire 64 is inserted through the guide 65 formed in an inner wall of the middle portion 165C and extends up to an operation part (not shown), provided at a region where the user's hand can approach, so as to be fixed to a handle (not shown). The tension wire 64 closer to the distal end than the guide 65 can be exposed from a notch (not shown) formed in an outer surface of the curved shape of the distal end portion 165A.

At the time when the suturing device or the suturing mechanism having the needle tube 165 with the above-described configuration is inserted into the channel of the endoscope device, the needle tube 165 is received in the sheath 40 without applying a pulling force to the tension wire 64. After the sheath 40 protrudes from the distal end of the endoscope device, when the needle tube 165 is made to protrude from the sheath 40 and the handle is pulled, the distal end portion 165A rotates about the second rotary pin 167 by means of the tension wire 64 and collides with an end surface of the middle portion 165C at an angle β to be held thereon. In the same manner, the middle portion 165C rotates about the first rotary pin 166 and collides with an end surface of the proximal end portion 165B at an angle α to be held thereon. In this manner, the needle tube 165 is deformed to the puncture shape by means of the user's operation.

In the above-described modified example, it is possible to maintain the needle tube 165 to be in a substantially rigid puncture shape by pulling the tension wire 64 via the handle, and to carry out the suturing operation with stronger force.

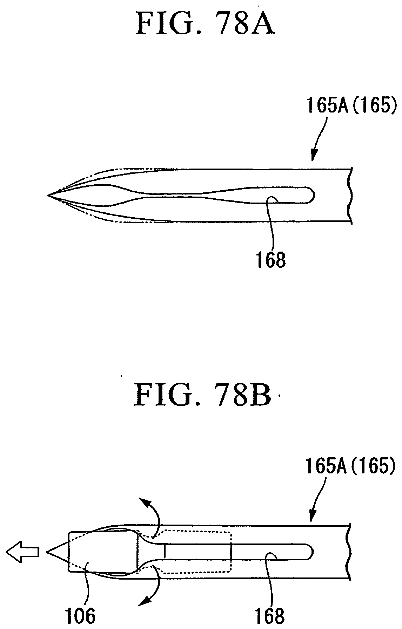

In the above-described modified example, as shown in FIG 78A, the needle tube may be formed so that both end portions of a groove 168, formed in substantially parallel at the distal end portion 165A of the needle tube 165, are curled inward until colliding with each other. With this structure, the distal end portion of the needle tube becomes sharper and the outer diameter becomes smaller, thereby easily puncturing the target tissue or the like with a small force.

As described above, since the needle tube 165 is made of an elastically deformable material, as shown in FIG 78B, the distal end portion of the groove 168 is instantly pushed to spread in accordance with a passage of the anchor upon releasing the anchor, thereby ejecting the anchor 106 or the like. After the passage of the anchor, the needle tube 165 is restored by means of elasticity so as to have a shape before the anchor is released.

As described above, the first to tenth embodiments are described, and an important common point in the above-described embodiments is that the material of the needle tube has both suitable elasticity and rigidity. In the case where the needle tube has such elasticity and rigidity, in addition to the super-elastic alloy mentioned so far, for example, a resin material such as polyamide, polyetheretherketone (PEEK), polysulfone, or liquid crystal polymer (LCP) or a biocompatible ceramic material such as alumina or silicon nitride may be appropriately adopted.

As described above, the preferred embodiments are described, but the invention is not limited to the embodiments. Additions, omissions, substitutions, and other modifications can be made without departing from the scope of the invention as defined in appended claim 1.

For example, in the above-described embodiments, there is described a case where the suturing unit is used in which the end portions of the suturing thread are mounted with two anchors, that is, the first anchor and the second anchor, but instead, the suturing unit may be used in which only one end portion of the suturing thread is mounted with one anchor.

In this case, the anchor penetrates two target tissues, facing each other with the treatment target portion interposed therebetween, at the same time so as to be locked thereon, the end portion of the suturing thread without the anchor is taken outside, and then an anchor is mounted to the end portion. Then, the anchor is introduced up to the treatment target portion along the suturing thread, and the suturing operation is carried out by means of the two anchors.

The invention is not limited to the foregoing description, but is limited by only the scope of the appended claims.

### INDUSTRIAL APPLICABILITY

According to the present invention, it is possible to observe the needle tube by means of the endoscope device when the needle tube is inserted into the target tissues or the like and after the needle tube has penetrated the tissue. It is possible to check that the distal end of the needle tube comes out from the target tissue or the like by means of the endoscope device. Also, it is possible to release the anchors while observing them by means of the endoscope device. Accordingly, it is possible to accurately carry out the suturing operation by reliably locking the suturing unit on the treatment target tissues.

### DESCRIPTION OF THE REFERENCE SYMBOLS

- 1, 31, 51, 51A, 161: SUTURING DEVICE
- 10, 35, 74: JAW
- 11, 39, 67, 162, 165: NEEDLE TUBE
- 13: PUSHER
- 32: GRIPPING MECHANISM
- 33: SUTURING MECHANISM
- 52: FIRST NEEDLE TUBE
- 53: SECOND NEEDLE TUBE
- 54: FIRST PUSHER
- 55: SECOND PUSHER
- 71, 91, 121, 131, 141, 151: SUTURING SYSTEM
- 77, 96: BENDING PORTION
- 78A, 78B, 97A, 97B: THROUGH-HOLE
- 80, 99: GUIDE SURFACE
- 92: SUCTION CAP
- 100: ENDOSCOPE DEVICE
- 103: SUTURING UNIT
- 132: PUNCTURE CAP
- 133: CYLINDRICAL BODY
- 133B: NEEDLE TUBE CHANNEL
- 142: LIFTING CAP
- 143: CAP SHEATH
- 145: LINK
- 152: CAP
- 155: THIN ENDOSCOPE

## Claims

1. A suturing system comprising:
a first endoscope (100) configured to observe a hollow organ (T1) and having a pair of channels along a longitudinal direction of the first endoscope (100);
a forceps (146) inserted in one of the pair of channels and configured to grip an edge of a perforation formed on the hollow organ (T1); and
a suturing mechanism (33) configured to suture tissues by using a suturing unit (103) having an anchor (106, 107) mounted to an end portion of a suturing thread (104), the suturing mechanism (33) including
a hollow needle tube (39) inserted in the other of the pair of channels, having an inner cavity capable of receiving the anchor (106, 107) to engage with a surrounding tissue of the perforation formed on the hollow organ (T1), and capable of penetrating the surrounding tissue in a state where the forceps (146) grips an edge of the perforation, and
a pusher (13) which is inserted through the hollow needle tube (39) in a freely advancing and retracting manner and is capable of pushing the anchor (106, 107) received in the hollow needle tube (39) toward a distal end of the hollow needle tube (39),
**characterized in that** the suturing system further comprises:
a second endoscope (155), mounted to the distal end portion of the first endoscope, disposed so that the hollow needle tube (39), the forceps (146) and the second endoscope (155) are arranged in series along a radial direction of the first endoscope (100), being configured to be inserted into the perforation formed on the hollow organ (T1), and configured to observe a state where the hollow needle tube (39) penetrates the surrounding tissue and protrudes therefrom, from the outside of the hollow organ (T1), wherein
the second endoscope (155) is capable of protruding to a more distal side than a distal end of the forceps (146) and the distal end of the hollow needle tube (39) and is capable of bending, and the second endoscope (155) is configured to observe the distal end of the hollow needle tube (39) by protruding and bending toward the distal end of the hollow needle tube (39).

## Patentansprüche

1. Nähsystem, das umfasst:
ein erstes Endoskop (100), das so konfiguriert ist, dass es ein Hohlorgan (T1) beobachtet, und das ein Paar von Kanälen entlang einer Längsrichtung des ersten Endoskops (100) aufweist;
eine Zange (146), die in einen des Paars von Kanälen eingeführt und so konfiguriert ist, dass sie einen Rand einer Perforation greift, die auf dem Hohlorgan (T1) gebildet ist; und
einen Nähmechanismus (33), der so konfiguriert ist, dass er Gewebe unter Verwendung einer Näheinheit (103) mit einem Anker (106, 107) näht, der an einem Endabschnitt eines Nähfadens (104) befestigt ist, wobei der Nähmechanismus (33) umfasst:
einen Hohlnadeltubus (39), der in den anderen des Paars von Kanälen eingeführt ist, mit einem Innenhohlraum, der in der Lage ist, den Anker (106, 107) aufzunehmen, um mit einem umliegenden Gewebe der auf dem Hohlorgan (T1) gebildeten Perforation in Eingriff zu gelangen, und der in der Lage ist, das umliegende Gewebe in einem Zustand, in dem die Zange (146) einen Rand der Perforation greift, zu penetrieren, und
einen Schieber (13), der durch den Hohlnadeltubus (39) eingeführt ist, um frei vorwärtsbewegt und zurückgezogen zu werden, und der in der Lage ist, den im Hohlnadeltubus (39) aufgenommenen Anker (106, 107) in Richtung eines distalen Endes des Hohlnadeltubus (39) zu schieben,
**dadurch gekennzeichnet, dass** das Nähsystem ferner umfasst:
ein zweites Endoskop (155), das am distalen Endabschnitt des ersten Endoskops befestigt ist, so angeordnet, dass der Hohlnadeltubus (39), die Zange (146) und das zweite Endoskop (155) entlang einer radialen Richtung des ersten Endoskops (100) in Reihe angeordnet sind, das so konfiguriert ist, dass es in die auf dem Hohlorgan (T1) gebildete Perforation eingeführt wird, und das so konfiguriert ist, dass es einen Zustand, in dem der Hohlnadeltubus (39) das umliegende Gewebe penetriert und von diesem hervorsteht, von außerhalb des Hohlorgans (T1) beobachtet, wobei
das zweite Endoskop (155) in der Lage ist, zu einer distaleren Seite als ein distales Ende der Zange (146) und das distale Ende des Hohlnadeltubus (39) vorzustehen, und in der Lage ist, sich zu biegen, und das zweite Endoskop (155) so konfiguriert ist, dass es das distale Ende des Hohlnadeltubus (39) durch Vorstehen und Biegen in Richtung des distalen Endes des Hohlnadeltubus (39) beobachtet.

## Revendications

1. Système de suture comprenant :
un premier endoscope (100) configuré pour observer un organe creux (T1) et présentant une paire de canaux le long d'une direction longitudinale du premier endoscope (100) ;
un forceps (146) inséré dans l'un de la paire de canaux et configuré pour saisir un bord d'une perforation formée sur l'organe creux (T1) ; et
un mécanisme de suture (33) configuré pour suturer des tissus à l'aide d'une unité de suture (103) ayant un ancrage (106, 107) monté sur une partie d'extrémité d'un fil de suture (104), le mécanisme de suture (33) comprenant :
un tube d'aiguille creux (39) inséré dans l'autre de la paire de canaux, ayant une cavité interne capable de recevoir l'ancrage (106, 107) pour venir en prise avec un tissu environnant de la perforation formée sur l'organe creux (T1), et capable de pénétrer dans le tissu environnant dans un état où le forceps (146) saisit un bord de la perforation, et
un poussoir (13) qui est inséré à travers le tube d'aiguille creux (39) d'une manière à avance et recul libres et est capable de pousser l'ancrage (106, 107) reçu dans le tube d'aiguille creux (39) vers une extrémité distale du tube d'aiguille creux (39),
**caractérisé par le fait que** le système de suture comprend en outre :
un second endoscope (155), qui est monté sur la partie d'extrémité distale du premier endoscope, est disposé de telle sorte que le tube d'aiguille creux (39), le forceps (146) et le second endoscope (155) sont agencés en série le long d'une direction radiale du premier endoscope (100), est configuré pour être inséré dans la perforation formée sur l'organe creux (T1), et est configuré pour observer un état où le tube d'aiguille creux (39) pénètre dans le tissu environnant et fait saillie à partir de celui-ci, depuis l'extérieur de l'organe creux (T1),
le second endoscope (155) étant capable de faire saillie sur un côté plus distal qu'une extrémité distale du forceps (146) et que l'extrémité distale du tube d'aiguille creux (39) et étant capable de se courber, et le second endoscope (155) étant configuré pour observer l'extrémité distale du tube d'aiguille creux (39) par saillie et courbure vers l'extrémité distale du tube d'aiguille creux (39).
